# EUROPEAN PATENT SPECIFICATION

(11) **EP 0 439 056 B1**
(45) Date of publication and mention of the grant of the patent: **13.04.1994**
(21) Application number: 91100502.3
(22) Date of filing: 17.01.1991
(51) Int. Cl.: C12N 15/30, A61K 39/012, C07K 13/00

(54) **Coccidiosis vaccines**
Vakzine gegen Coccidiose
Vaccin contre la coccidiose

(30) Priority: 26.01.1990 US 470508
(43) Date of publication of application: 31.07.1991
(73) Proprietor: F. HOFFMANN-LA ROCHE AG, 4002 Basel (CH)
(72) Inventor: Binger, Mary-Helen, Hopewell, N.J. 08525 (US)
(74) Representative: Mezger, Wolfgang, Dr.

(56) References cited:
- EP-A- 0 135 073
- EP-A- 0 164 176
- EP-A- 0 344 808
- "Molecular Cloning" J. Sambrook et al., 1989 pages 18.47-18.48 Cold Spring Harbour Laboratory Press

## Description

This application relates to antigens of Eimeria protozoan parasites. These antigens can be used, through various routes of administration, to protect poultry against coccidiosis.

Coccidiosis is a disease of poultry caused by intracellular protozoan parasites of the genus Eimeria. The disease is endemic in large, intensive poultry breeding establishments. The estimated cost of control of the disease through chemotherapy exceeds $100 million each year in the United States of America alone. The development of resistance to the known anti-coccidial drugs necessitates a continuing development of new agents, at a time when drug development is becoming increasingly expensive and consumer acceptance of drug residues in food animals is diminishing.

Protective immunity to natural coccidiosis infection has been well documented. Controlled, daily administration of small numbers of viable oocysts for several weeks has been shown to result in complete immunity to a challenge infection of a normally virulent dose [Rose et al., Parasitology 73:25 (1976); Rose et al., Parasitology 88:199 (1984)]. The demonstration of acquired resistance to infection suggests the possibility of constructing a vaccine to induce immunity in young chickens, circumventing the need for chemical coccidiostats. In fact, such a concept has been tested in the Coccivac @ formulation of Sterwin Laboratories, Opelika, AL.

With a view to producing a coccidiosis vaccine, Murray et al., European Patent Application, Publication No. 167,443, prepared extracts from sporozoites or sporulated oocysts of Eimeria tenella which contain at least 15 polypeptides, many of which were associated with the surface of the sporozoite. Injection of these extracts into chickens reduced cecal lesions following oral inoculation with virulent E. tenella sporulated oocysts.

More recently, Schenkel et al., U.S. Patent No. 4,650,676, disclosed the production of monoclonal antibodies against E. tenella merozoites. Using these antibodies, Schenkel et al. identified a number of antigens against which the antibodies were directed. By pre-incubating E. tenella sporozoites with these antibodies and then introducing the treated sporozoites into the ceca of chickens, Schenkel et al. were able to show some reduction in cecal lesion scores, compared to untreated sporozoite controls.

Using recombinant DNA methodology, Newman et al. (European Patent Application, Publication No. 164 176) have cloned a gene from the sporozoite stage coding for a 25,000 dalton antigen from Eimeria tenella. Sera from chickens immunized by repeated immunization with killed E. tenella sporozoites immunoprecipitated this antigen from iodinated sporocyst and sporozoite membrane preparations. More recently, Jenkins [Nucleic Acids Res. 16:9863 (1988)] has described a cDNAencoding a part of a 250,000 dalton merozoite surface protein from Eimeria acervulina. The expression product of this cDNAwas recognized by antiserum against the organism.

Advances in recombinant DNA technology have made another approach available, i.e. a subunit vaccine. Examples of such subunit vaccines are described e.g. in European Patent Application, Publication Nos. 324 648, 337 589 and 344 808.

The present invention provides a protein having one or more immunoreactive and/or antigenic determinants of an Eimeria merozoite surface antigen, which surface antigen has an apparent molecular weight of about 23 kilodaltons by sodium dodecylsulfate polyacrylamide gel electrophoresis (SDS PAGE) and is derived from a precursor protein having an apparent molecular weight of about 30 kilodaltons by SDS PAGE and which protein is encoded by the nucleotide sequence 
ATGGCTAAGTCTATGCTTTCTGGAATTGTTTTTGCTGGTCTTGTTGCTGCTGCAGCGGCC
AGTTCGGCCAACAGCGCCGCCAACGTCTCCGTTTTGGAGAGTGGGCCCGCTGTGCAGGAA
GTGCCAGCGCGCACGGTCACAGCTCGCCTGGCGAAGCCTTTGCTGCTTCTTTCTGCTCTT
GCTGCGACTTTGGCAGCAGCTTTCCTCGTTTTGCAATGCTTCAACACCATCTCCAGCAAC
AACCAGCAAACCAGCGTCAGGAGACTGGCCGCCGGAGGTGCATGCGGAGATGAGGAAGAT
GCAGATGAGGGAACTTCACAGCAGGCCAGCCGGAGGAGGAGAAAACCTGATACCCCTGCA
GCAGATAAATACGATTTTGTTGGCGGAACTCCAGTTTCGGTCACTGAGCCGAATGTTGAT
GAAGTCCTTATCCAAATTAGAAATAAACAAATCTTTTTGAAGAACCCATGGACTGGACAA
GAAGAACAAGTTCTAGTACTGGAACGACAAAGTGAAGAACCCATTCTCATTGTGGCGAGG
ACAAGACAACACTTGAAGGATATCTTGGTAGTCAGCTCTTGCACAGGACGGAAAGACTGC
TAA
 or an equivalent sequence thereof, said protein being free of other Eimeria proteins.

More particularly, this invention provides an isolated protein which is the Eimeria merozoite surface antigen having an apparent molecular weight of about 23 kilodaltons determined by SDS PAGE and fragments of the said protein. These proteins and fragments are free of other Eimeria proteins.

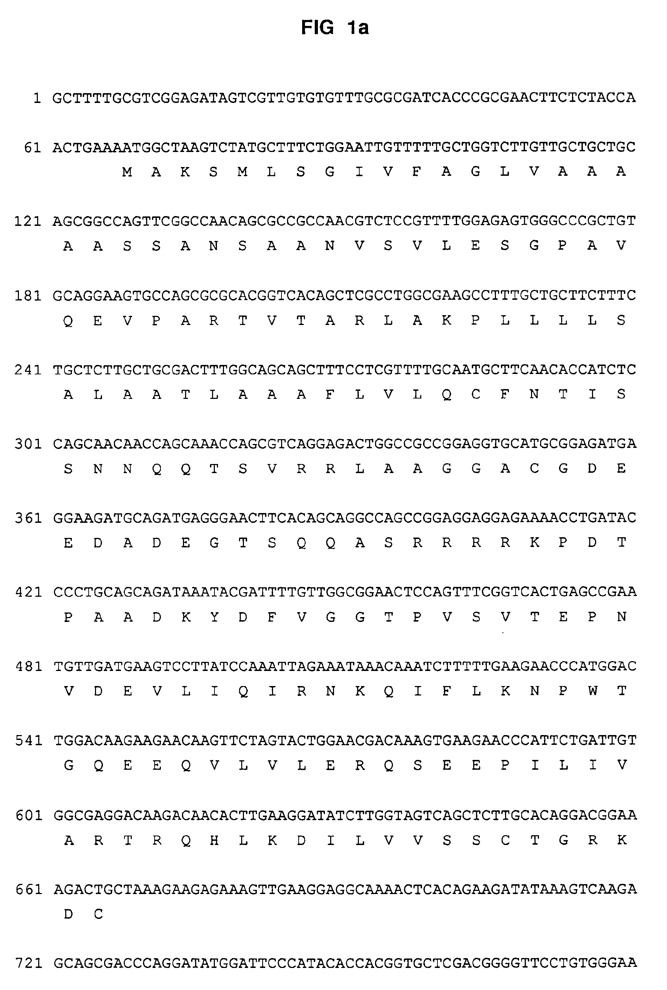

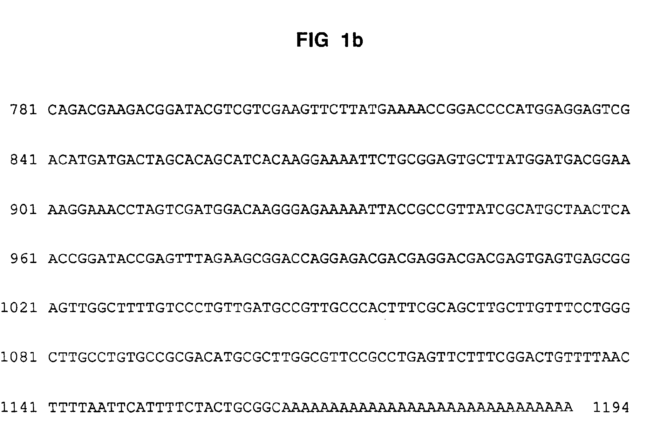

This invention further provides a protein which is the precursor protein to the Eimeria merozoite surface antigen mentioned above, or a fragment thereof, which precursor protein has an apparent molecular weight of about 30 kilodaltons determined by SDS PAGE and has the amino acid sequence shown in Figure 1. The said precursor protein is free of other Eimeria proteins.

The preferred protein of the present invention is the mature Eimeria merozoite surface antigen protein having the amino acid sequence shown in Figure 1 but lacking the signal peptide sequence at the N-terminus, which signal peptide sequence comprises the first twenty amino acids in the sequence shown in Figure 1. The present invention also relates to a functional equivalent protein thereof having an amino acid sequence which is related to the said amino acid sequence by deletions, insertions or substitutions without changing the immunological properties of the said protein.

This invention still further provides a DNA encoding all or part of the Eimeria merozoite surface antigen having an apparent molecular weight of about 23 kilodaltons or its above-mentioned precursor protein, recombinant vectors containing and capable of directing the expression of the said DNAin compatible host organisms, and microorganisms containing such vectors.

This invention still further provides a method for producing a protein having one or more immunoreactive and/or antigenic determinants of an Eimeria merozoite surface antigen which surface antigen has an apparent molecular weight of about 23 kilodaltons, which method comprises:
(a) culturing a microorganism containing a recombinant vector comprising a DNA having a nucleotide sequence encoding the said protein such as the DNA having the nucleotide sequence depicted in Figure 1 or a fragment thereof, under conditions in which the DNA sequence or fragment is expressed; and
(b) isolating the protein from the culture.

This invention still further provides vaccines for protecting poultry against coccidiosis comprising an effective amount of one or more of the proteins of the invention and a physiologically acceptable carrier.

This invention still further provides vaccines for protecting poultry against coccidiosis comprising a recombinant virus containing a DNA sequence encoding a protein of the present invention, which recombinant virus is capable of causing the expression of the said DNA sequence, and a physiologically acceptable carrier.

This invention still further provides a method for protecting poultry against coccidiosis, which method comprises administering an effective amount of a vaccine of the invention to a young fowl which is susceptible to coccidiosis.

The Eimeria proteins of this invention are important vaccine antigens because they were identified by the use of antibodies in the sera of animals that had been immunized against the coccidiosis organism and had developed immunity thereto. Because of this, it is most likely that these proteins play a significant role in the protection of poultry against coccidiosis.

The invention can be more readily understood by reference to the figures, in which:
Fig. 1 shows the nucleotide sequence of the 1.2 kb cDNA molecule encoding the Eimeria precursor protein recognized by antibody-select antibodies from rabbit and by chicken immune sera. As can be seen from Fig. 1, the nucleotide sequence encoding the said precursor protein is contained between the ATG at nucleotide 68 and the stop codon TAA at nucleotide 668 (coding for 200 amino acids). Fig. 1 also shows the amino acid sequence of the Eimeria precursor protein predicted from the nucleotide sequence provided. Standard single- letter abbreviations are used to represent nucleotides and amino acids. The meanings of these abbreviations can be found in standard biochemistry textbooks, such as Lehninger, Principles of Biochemistry, 1984, Worth Publishers, Inc., New York, pp. 96, 798.

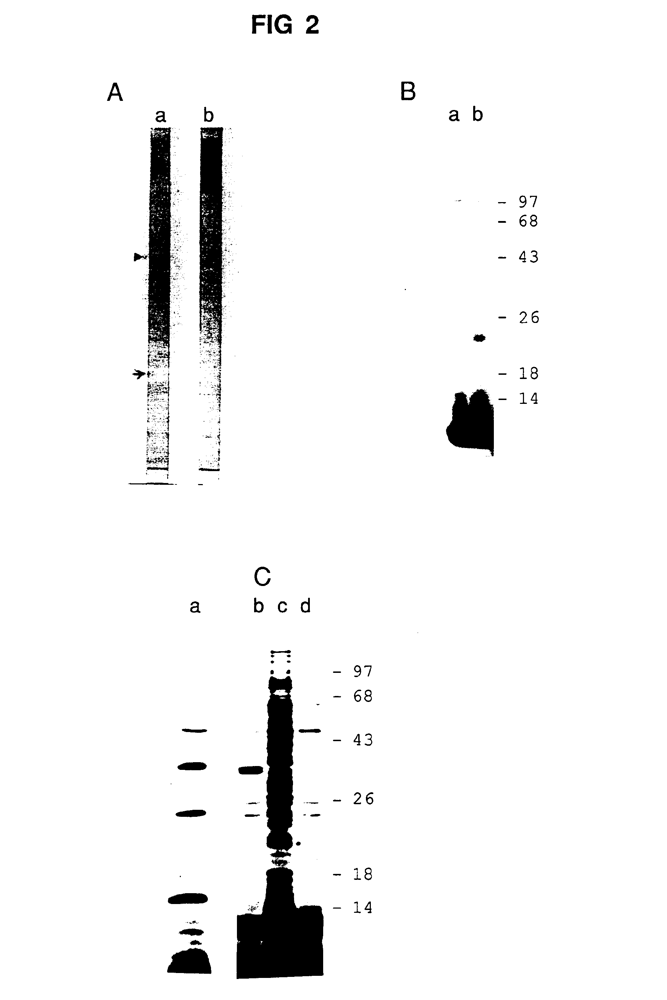

Fig. 2 shows the results of an SDS PAGE analysis of various Eimeria merozoite proteins. Panel A is an immunoblot of total merozoite proteins probed with control (a) or antibody-select (b) antibodies. The arrow in Panel A indicates the position of a band containing a protein having molecular weight of about 23 kilodaltons. Panel B is an autoradiogram of ¹²⁵1-surface-labeled merozoite proteins immunoprecipitated with control (a) or antibody-select (b) antibodies. Panel C shows the complete mixture of products produced by the in vitro translation of merozoite mRNA (a) and translation products which had been immunoprecipitated with antibodies selected using the lambda 5-7 clone (b), antibodies selected using another phage clone which produced proteins reactive with anti-merozoite serum (c) and control antibodies selected from merozoite serum using non-recombinant phage (d). The bands were visualized by fluorography. The positions of molecular weight markers having the indicated molecular weight in kilo Daltons (kDa) are shown to the right of the figure.
Fig. 3 shows the results of Southern Blot analysis of Eimeria tenella sporulated oocyst genomic DNAwhich has been digested with Pvull (lane 1), Hincll (lane 2), Pstl (lane 3), Sphl (lane 4) or Sacl (lane 5). The positions of standard DNAs having the indicated sizes in kb are shown to the right of the figure.

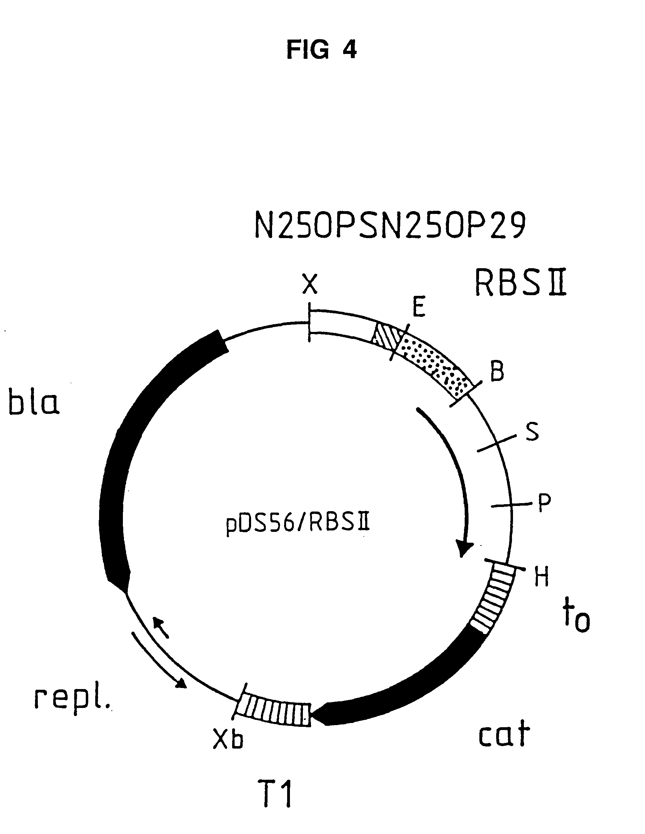

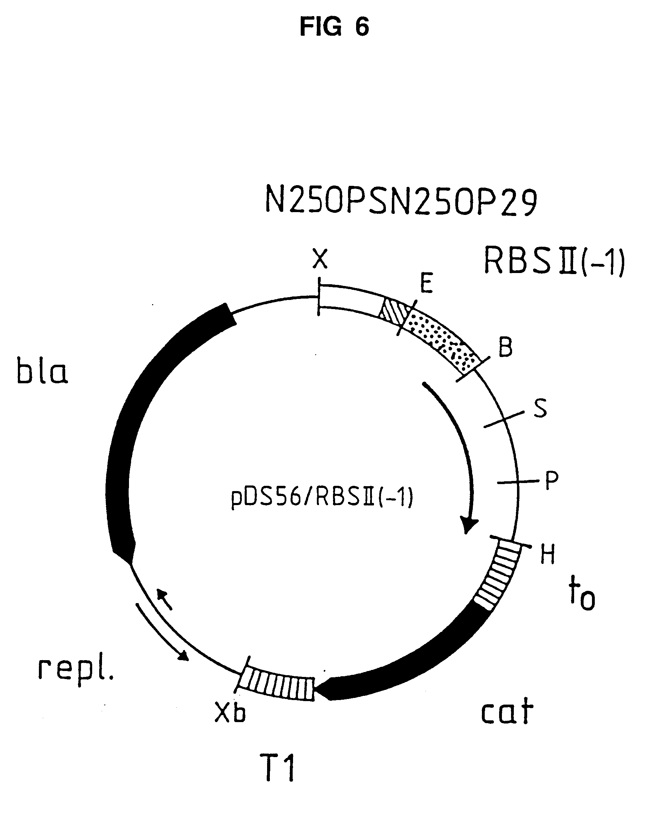

Fig. 4 shows a schematic drawing of the plasmid pDS56/RBSII. In this diagram and in Figs. 6, 8 and 10, the abbreviations and symbols B, Bg, E, H, N, P, S, X and Xb indicate cleavage sites for restriction enzymes BamHl, Bglll, EcoRl, Hindlll, Ncol, Pstl, Sall, Xhol and Xbal, respectively. 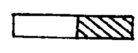 represents the regulatable promoter/operator element N250PSN25OP29; 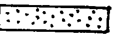 represents ribosomal binding sites RBSII, RBSII(-1) and RBSII(-2); → represents coding regions under control of these ribosomal binding sites; 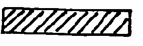 represents a region encoding six histidine residues; 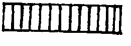 repre- ← sents terminators to and T1; → represents the region required for DNA replication in E. coli (repl.); 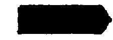 represents coding regions for dihydrofolate reductase (dhfr), chloramphenicol acetyltransferase (cat), (3-lactamase (bla), lac repressor (lacl) and neomycin phosphotransferase (neo).
Fig. 5 displays the complete nucleotide sequence of the plasmid pDS56/RBSII. In this sequence, the recognition sequences of the restriction enzymes depicted in Fig. 4 are indicated. The amino acid sequence shown represents the open reading frame under control of ribosomal binding site RBSII.
Fig. 6 is a schematic drawing of the plasmid pDS56/RBSII(-1).
Fig. 7 displays the complete nucleotide sequence of plasmid pDS56/RBSII(-1). In this sequence, the recognition sequences of the restriction enzymes depicted in Fig. 6 are indicated. The amino acid sequence shown represents the open reading frame under control of ribosomal binding site RBSII(-1).
Fig. 8 is a schematic drawing of the plasmid pDS56/RBSII(-2).
Fig. 9 displays the complete nucleotide sequence of plasmid pDS56/RBSII(-2). In this sequence, the recognition sequences of the restriction enzymes depicted in Fig. 8 are indicated. The amino acid sequence shown represents the open reading frame under control of ribosomal binding site RBSII(-2).
Fig. 10 is a schematic drawing of the plasmid pDMI.1.
Fig. 11 displays the complete nucleotide sequence of plasmid pDMI.1. In this sequence, the recognition sequences of the restriction enzymes depicted in Fig. 10 are indicated. The amino acids shown enclose the open reading frames encoding the neomycin phosphotransferase (Met to Phe) and the lac repressor (Met to Gin; please note the reverse orientation of this gene).

All references cited herein are hereby incorporated in their entirety by reference.

As used herein, the following terms shall have the following meanings:
"Eimeria surface antigen" means a protein having an apparent molecular weight of about 23 kilodaltons in SDS PAGE which is present in the merozoite stage of Eimeria tenella. This protein appears to be produced by post-translational processing of the in vivo expression product of a gene having the nucleotide sequence shown in Fig. 1.

"Precursor protein" means a protein having an apparent molecular weight of about 30 kilodaltons in SDS PAGE. This protein is believed to be processed by proteolysis in vivo to the Eimeria surface antigen. The nucleotide sequence of a cDNAmolecule encoding this protein and the amino acid sequence predicted therefrom are shown in Fig. 1.

The term "a protein having one or more immunoreactive and/or antigenic determinants of the Eimeria surface antigen" means a protein having one or more regions or epitopes which are capable of eliciting an immune response in an immunologically competent host organism and/or are capable of specifically binding to a complementary antibody, and which correspond to the epitopes of the Eimeria surface antigen defined above. The said protein may be encoded by functional equivalents of the nucleotide sequence of Fig. 1. These functional equivalent proteins have amino acid sequences related to the sequence of Fig. 1 by amino acid substitutions which do not substantially alter immunological activity (i.e., which do not substantially destroy immunoreactive and/or antigenic determinants).

Because of the degeneracy of the genetic code, it will be understood that there are many potential nucleotide sequences (functional equivalents) that could code for the amino acid sequence shown in Fig. 1. It should also be understood thatthe nucleotide sequences of the DNAsequences and fragments of the invention inserted into vectors may include nucleotides which are not part of the actual structural genes, as long as the recombinant vectors containing such sequence or fragments are capable of directing the production in an appropriate host organism of a protein or fragment having one or more immunoreactive and/or antigenic determinants of the Eimeria surface antigen.

Amino acid substitutions in proteins which do not substantially alter biological and immunological activities have been known to occur and have been described, e.g., by Neurath et al., in "The Proteins", Academic Press, New York (1979), in particular in Fig. 6 at page 14. The most frequently observed amino acid substitutions are Ala/Ser, Val/lie, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Glu, Asp/Gly, and vice versa.

Such functionally equivalent nucleotide sequence variations and amino acid substitutions of the exemplary embodiments of this invention are within the scope of the invention as long as the resulting proteins retain one or more immunoreactive and/or antigenic determinants of the Eimeria surface antigen as herein defined.

Other DNA sequences encoding the amino acid sequence of Fig. 1 or amino acid sequences related by substitutions can readily be prepared using appropriate synthetic oligonucleotides in primer-directed site- specific mutagenesis on the exemplary cDNA of this invention (Fig. 1), as described by Morinaga et al. [Biotechnology 2:636 (1984)].

The term "fragment" means an oligonucleotide or polypeptide comprising a sub-sequence of one of the cDNAs or proteins of the invention. Such fragments can be produced by enzymatic cleavage of the larger molecules, using restriction endonucleases for the DNA and proteases for the proteins. The fragments of the invention, however, are not limited to the products of any form of enzymatic cleavage but include sub-sequences, the termini of which do not correspond to any enzymatic cleavage points. Such fragments can be made, e.g., by chemical synthesis, using the sequence data provided herein. DNA fragments can also be produced by incomplete complementary DNA (cDNA) synthesis from isolated messenger RNA (mRNA). Protein fragments can also be produced by expressing DNA fragments encoding the protein fragments. Such protein fragments can be useful in the present invention if they contain a sufficient number of amino acid residues to constitute an immunoreactive and/or antigenic determinant. Generally, at least about 7 or 8 residues are needed. As explained below, it may be necessary to couple such fragments to an immunogenic carrier molecule, to make them immunoreactive.

The proteins of this invention can be made by methods known in the art such as by recombinant DNAmeth- odology, chemical synthesis or by isolation from Eimeria preparations.

DNA needed to make the proteins of this invention could be chemically synthesized, using the nucleotide sequence information provided in Fig. 1. Such chemical synthesis can be carried out using any of the known methods such as the phosphoramidite solid support method of Matteucci et al. [J. Am. Chem. Soc. 103:3185 (1981)].

Alternatively, cDNAcan be made from Eimeria mRNA. Messenger RNAcan be isolated from Eimeria merozoites using standard techniques. These mRNA samples can be used to produce double-stranded cDNA as described by Maniatis et al. [Molecular Cloning: A Laboratory Manual, 1982, Cold Spring Harbor Laboratory, Cold Spring Harbor, NY]. This cDNAcan then be inserted into an appropriate cloning vector which can be used to transform E. coli, to produce a cDNA library.

The cDNA library can then be screened using the cloned gene of this invention, or fragments thereof, as probes. Such gene or fragments can be radiolabeled, e.g., by nick-translation using Pol I DNA polymerase in the presence of the fourdeoxyribonucleotides, one of which contains ³²P in the a position (Maniatis et al., supra, p. 109), for use as probes. The probes may also be prepared by oligonucleotide synthesis based on the known sequence of the cDNA of the Eimeria surface antigen.

Although Eimeria tenella was used as an mRNA source in the Examples below, the cloned genes from this species can be used as probes to isolate genes from other species of Eimeria, due to DNAsequence homology among the various species.

Once identified and isolated, the Eimeria DNAsequences of this invention are inserted into an appropriate expression vehicle which contains the elements necessary for transcription and translation of the inserted gene sequences. Useful cloning vehicles may consist of segments of chromosomal, nonchromosomal and synthetic DNA sequences such as various known bacterial plasmids, phage DNA, combinations of plasmids and phage DNAsuch as plasmids which have been modified to employ phage DNA or other expression control sequences, or yeast plasmids. Specific cloning vehicles which could be used include but are not limited to the pEV-vrf plasmids (pEV-vrf1, -2 and -3 which are described in Crowl et al., Gene 38:31 (1985)); SV40; adenovirus; yeast; lambda gt-WES-lambda B; Charon 4A and 28; lambda-gt-l-lambda B; M13-derived vectors such as pUC8, 9, 18 and 19, pBR313, 322 and 325; pAC105; pVA51; pACY177; pKH47; pACYC184; pUB110; pMB9; colE1; pSC101; pML21; RSF2124; pCR1 or RP4; fowipox; vaccinia; a member of the herpesvirus family.

The insertion of the Eimeria genes into a cloning vector is easily accomplished when both the genes and the desired cloning vehicle have been cut with the same restriction enzyme or enzymes, since complementary DNAtermini are thereby produced. If this cannot be accomplished, it may be necessary to modify the cut ends that are produced by digesting back single-stranded DNAto produce bluntends, or by achieving the same result by filling in the single-stranded termini with an appropriate DNA polymerase. In this way, blunt-end ligation with an enzyme such as T4 DNA ligase may be carried out. Alternatively, any site desired may be produced by ligating nucleotide sequences (linkers) onto the DNA termini. Such linkers may comprise specific oligonucleotide sequences that encode restriction site recognition sequences. The cleaved vector and the Eimeria genes or fragments may also be modified by homopolymeric tailing, as described by Morrow [Methods in Enzymology 68:3 (1979)].

Many of the cloning vehicles that may be used in this invention contain one or more marker activities that may be used to select for desired transformants, such as ampicillin and tetracycline resistance in pBR322, ampicillin resistance and β-galactosidase activity in pUC8, and ampicillin resistance in the pEV-vrf plasmids. Selection of host cells into which such vectors have been inserted is greatly simplified when the host cells otherwise lack the activities contributed by the vectors.

It should be understood that the nucleotide sequences of the Eimeria genes inserted at a selected site in a cloning vehicle may include nucleotides which are not part of the actual structural genes. Alternatively, the genes may contain only part of the complete wild-type gene. All that is required is that the gene fragments after insertion into a cloning vehicle are capable of directing the production in an appropriate host organism of a polypeptide or protein having at least one immunoreactive and/or antigenic determinant of the Eimeria surface antigen. Thus, the recombinant vectors comprising a DNA having a nucleotide sequence encoding a protein of the present invention may be prepared by:
(a) inserting a DNA having a nucleotide sequence encoding the said protein into a vector;
(b) replicating the said vector in a microorganism; and
(c) isolating the recombinant vector from the microorganism.

The selection of an appropriate host organism is affected by a number of factors known in the art. These factors include, for example, compatibility with the chosen vector, toxicity of proteins encoded by the hybrid plasmid, ease of recovery of the desired protein, expression characteristics, biosafety and costs. A balance of these factors must be considered, and it must be understood that not all hosts will be equally effective for expression of a particular recombinant DNA molecule.

Suitable host microorganisms which can be used in this invention include but are not limited to plant, mammalian or yeast cells and bacteria such as Escherichia coli, Bacillus subtilis, Bacillus stearothermophilus and Actinomyces. Escherichia coli strain MC1061, which has been described by Casadaban et al. [J. Mol. Biol. 138:179 (1980)], can be used, or any other strain of E. coli K-12 containing the plasmid pRK248clts. Plasmid pRK248clts for use in other E. coli K-12 strains is described by Bernhard et al. [Meth. of Enzymol. 68:482 (1979)] and is also available from the American Type Culture Collection under accession No. ATCC 33766. The E. coli strain MC1061 is commercially available e.g. from CLONTECH Laboratories, Inc., Palo Alto, CA and is also available from the American Type Culture Collection under accession No. ATCC 53338. Plasmids pDM1.1, pDS56/RBSII, -1 or -2 for use in E. coli strain M15 are described infra.

Transfer of the recombinant cloning vector into the host cell may be carried out in a variety of ways. Depending upon the particular vector/host cell system chosen, such transfer may be effected by transformation, transduction or transfection. Once such a modified host cell is produced, the cell can be cultured and the protein expression product may be isolated from the culture.

Transformant clones producing the precursor protein of the Eimeria surface antigen are identified by screening with serum from animals immunized against glutaraldehyde-fixed sporozoites or merozoites of E. tenella. In the examples below, rabbit anti-merozoite serum was used for screening and characterizing the gene product. Parallel immunlogic screening with immune chicken serum resulted in the independent isolation of the cDNA encoding the merozoite surface antigen.

The specificity of the antisera used for immunological screening or immunoprecipitation can be increased by using a variation of the antibody select method of Hall et al. [Nature 311:379 (1984)]. In this method, which is described more fully below, antibodies that are specific for Eimeria proteins made by the clones are adsorbed out on filters.

The detection of Eimeria antigen producing clones can be achieved by the use of well known standard assay methods, including immunoprecipitation, enzyme-linked immunoassay and radioimmunoassay techniques which have been described in the literature [see, e.g., Kennet et al. (editors), Monoclonal Antibodies and Hybridomas: A New Dimension in Biological Analyses, 1980, Plenum Press, New York, pp. 376-384].

Large amounts of the recombinant Eimeria protein may be produced by growing the transformed microorganisms obtained in this way in a fermentation broth comprising the necessary nutrients under conditions suitable for expression of the recombinant DNA. As produced in E. coli, the recombinant Eimeria proteins are in the cytoplasm or in inclusion bodies. To free the proteins it is thus necessary to disrupt the outer membrane of the bacteria. This is accomplished by sonication, or by other mechanically disruptive means, such as by using a French pressure cell or Gaulin homogenizer [Charm et al., Meth. Enzymol. 22, 476-556 (1971)].

Cell disruption can also be accomplished by chemical or enzymatic means. Since divalent cations are often required for cell membrane integrity, treatment with appropriate chelating agents such as EDTAor EGTAmight prove sufficiently disruptive to facilitate the leakage of the proteins from the cells. Similarly, enzymes such as lysozyme have been used to achieve the same result. That enzyme hydrolyzes the peptidoglycan backbone of the cell wall.

The application of osmotic shock can also be employed. Briefly, this can be accomplished by first placing the cells in a hypertonic solution which would cause them to lose water and shrink. Subsequent placement in a hypotonic "shock" solution would then lead to a rapid influx of water into the cells with an expulsion of the desired proteins.

Once freed from the cells, the Eimeria proteins may be concentrated by precipitation with salts such as sodium or ammonium sulfate, ultrafiltration or other methods well known to those skilled in the art. Further purification could be accomplished by conventional protein purification techniques including but not limited to gel filtration, ion-exchange chromatography, preparative disc-gel or curtain electrophoresis, isoelectric focusing, low temperature organic solvent fractionation, or countercurrent distribution. Purification can also be carried out by immunoaffinity chromatography.

Specific methods for purifying Eimeria proteins from the organisms are known in the art. See, e.g., Newman et al., European Patent Application, Publication No. 164 176.

The proteins of this invention orfragments thereof can also be chemically synthesized by a suitable method such as by exclusive solid phase synthesis, partial solid phase methods, fragment condensation or classical solution synthesis. Solid phase synthesis as described by Merrifield [J. Am. Chem. Soc. 85:2149 (1963)] is preferred.

Such synthesis is carried out with amino acids that are protected at the alpha-amino-terminus. Trifunctional amino acids with labile side-chains are also protected with suitable groups which will prevent a chemical reaction from occurring at that site during the assemblage of the peptide. The alpha-amino protecting group is selectively removed to allow subsequent reaction to take place at the amino-terminus. The conditions for the removal of the alpha-amino protecting group do not cause deprotection of the side-chain protecting groups.

The alpha-amino protecting groups are those known to be useful in the art of stepwise synthesis of peptides. Included are acyl type protecting groups (e.g., formyl, trifluoroacetyl, acetyl), aromatic urethane type protecting groups (e.g., benzyloxycarbonyl (Cbz) and substituted benzyloxycarbonyl), aliphatic urethane protecting groups (e.g., t-butyloxycarbonyl (Boc), isopropyloxycarbonyl, cyclohexyloxycarbonyl) and alkyl type protecting groups (e.g., benzyl, triphenylmethyl). The preferred protecting group is Boc. The side-chain protecting groups for Tyr include tetrahydropyranyl, tert.-butyl, triyl, benzyl, Cbz, 4-Br-Cbz and 2,6-dichlorobenzyl. The preferred side-chain protecting group for Tyr is 2,6-dichlorobenzyl. The side-chain protecting groups for Asp include benzyl, 2,6-dichlorobenzyl, methyl, ethyl and cyclohexyl. The preferred side-chain protecting group for Asp is cyclohexyl. The side-chain protecting groups for Thr and Ser include acetyl, benzoyl, trityl, tetrahydropyranyl, benzyl, 2,6-dichlorobenzyl and Cbz. The preferred protecting group for Thr and Ser is benzyl. The side-chain protecting groups for Arg include nitro, Tos, Cbz, adamantyloxycarbonyl or Boc. The preferred protecting group for Arg is Tos. The side-chain amino group of Lys may be protected with Cbz, 2-CICbz, Tos or Boc. The 2-CI-Cbz group is the preferred protecting group for Lys. The selection of the side-chain protecting group is based on the following: The side-chain protecting group remains intact during coupling and is not split off during the deprotection of the amino-terminus protecting group or during coupling conditions. The side-chain protecting group must be removable upon the completion of the synthesis of the final peptide, using reaction conditions that will not alter the target peptide.

Solid phase synthesis is usually carried out from the carboxy-terminus by coupling the alpha-amino protected (side-chain protected) amino acid to a suitable solid support. An ester linkage is formed when the attachment is made to a chloromethylated or hydroxymethyl resin and the resultant target peptide will have a free carboxyl group at the C-terminus. Alternatively, a benzhydrylamine or p-methylbenzhydrylamine resin is used in which case an amide bond is formed and the resultant target peptide will have a carboxamide group at the C-terminus. These resins are commercially available and their preparation is described by Stewart et al., "Solid Phase Peptide Synthesis" (2nd Edition, Pierce Chemical Co., Rockford, IL., 1984).

The C-terminal amino acid, Arg, protected at the side-chain with Tos and at the alpha-amino function with Boc is coupled to the benzhydrylamine resin using various activating agents including dicyclohexylcarbodiimide (DCC), N,N'-diisopropylcarbodiimide and carbonyldiimidazole. Following the attachment to the resin support the alpha-amino protecting group is removed by using trifluoroacetic acid (TFA) or HCI in dioxane at a temperature between 0° and 25°C. Dimethylsulfide is added to the TFA after the introduction of methionine (Met) to suppress possible S-alkylation. After removal of the alpha-amino protecting group, the remaining protected amino acids are coupled stepwise in the required order to obtain the desired peptide sequence.

Various activating agents can be used for the coupling reactions including DDC, N,N'-diisopropylcarbodiimide, benzotriazol-1-yl-oxy-tris-(dimethylamino)-phosphonium hexafluorophosphate (BOP) and DCC-hydroxybenzotriazole (HOBt). Each protected amino acid is used in excess (>2.5 equivalents), and the couplings are usually carried out in DMF, CH₂CI₂ or mixtures thereof. The extent of completion of the coupling reaction is monitored at each stage by the ninhydrin reaction as described by Kaiser et al. [Anal. Biochem. 34:595 (1970)]. In cases where incomplete coupling is determined the coupling reaction is repeated. The coupling reactions can be performed automatically on a Vega 250, Applied Biosystems synthesizer or other commercially available instrument. Afterthe entire assemblage of the target peptide, the peptide-resin is deprotected with TFA/di- thioethane and then cleaved with a reagent such as liquid HF for 1-2 hours at 0°C which cleaves the peptide from the resin and removes all side-chain protecting groups.

Side-chain to side-chain cyclization on the solid support requires the use of an orthogonal protection scheme which enables selective cleavage of the side-chain functions of the acidic amino acids (e.g., Asp) and the basic amino acids (e.g., Lys). The 9-fluorenylmethyl (OFm) protecting group for the side-chain of Asp and the 9-fluorenylmethoxycarbonyl (Fmoc) protecting group for the side-chain of Lys can be used for this purpose. In these cases the side-chain protecting groups of the Boc-protected peptide-resin are selectively removed with piperidine in DMF. Cyclization is achieved on the solid support using various activating agents including DCC, DCC/HOBt or BOP. The HF reaction is carried out on the cyclized peptide-resin as described above.

Purification of the synthetic proteins can be carried out as described above for the recombinantly produced proteins.

Eimeria proteins can also be recovered from the organisms, from extracts of membrane proteins. Such methods can produce the complete, wild-type proteins. Monoclonal antibodies for this purpose can be produced as described by Köhler and Milstein [Nature 256:495 (1975)], using synthetic or natural Eimeria proteins as the antigen. These methods can be used to purify the 23 kd Eimeria surface antigen of this invention.

One or more of the Eimeria proteins of this invention can be formulated into vaccines comprising the proteins and a physiologically acceptable carrier. Suitable carriers include, e.g., 0.01 to 0.1 M phosphate buffer of neutral pH or physiological saline solution.

Enhanced immunity against coccidiosis can be produced in one of two ways. First, an adjuvant or immu- nopotentiator can be added to the vaccine. Secondly, the proteins of the invention can be presented to an animal that is to be immunized in a larger form, either as a cross-linked complex or conjugated to a carrier molecule.

Suitable adjuvants for the vaccination of animals include but are not limited to Adjuvant 65 (containing peanut oil, mannide monooleate and aluminum monostearate); mineral gels such as aluminum hydroxide, aluminum phosphate and alum; surfactants such as hexadecylamine, octadecylamine, lysolecithin, dimethyldiocta- decylammonium bromide, N,N-dioctadecyl-N',N'-bis(2-hydroxymethyl) propanediamine, methoxyhexadecyl- glycerol and pluronic polyols; polyanions such as pyran, dextran sulfate, poly IC, polyacrylic acid and carbopol; peptides such as muramyl dipeptide, dimethylglycine and tuftsin; and oil emulsions. The proteins could also be administered following incorporation into liposomes or other microcarriers.

Incorporation into liposomes or other microcarriers provides a means by which the release of the vaccines can be sustained over a prolonged period of time. A pump such as an Alza osmotic pump could be used for the same purpose.

The immunogenicity of the proteins of the invention, especially the smaller fragments, can be enhanced by cross-linking or by coupling to an immunogenic carrier molecule (i.e., a macromolecule having the property of independently eliciting an immunological response in a host animal, to which the proteins and protein fragments of the invention can be covalently linked). Cross-linking or conjugation to a carrier molecule may be required because small protein fragments sometimes act as haptens (molecules which are capable of specifically binding to an antibody but incapable of eliciting antibody production, i.e., they are not immunogenic). Conjugation of such fragments to an immunogenic carrier molecule renders the fragments immunogenic through what is commonly known as the "carrier effect".

Suitable carrier molecules include, e.g., proteins and natural or synthetic polymeric compounds such as polypeptides, polysaccharides, lipopolysaccharides etc. A useful carrier is a glycoside called Quil A, which has been described by Morein et al. [Nature 308:457 (1984)]. Protein carrier molecules are especially preferred, including but not limited to mammalian serum proteins such as keyhole limpet hemocyanin, human or bovine gammaglobulin, human, bovine or rabbit serum albumin, or methylated or other derivatives of such proteins. Other protein carriers will be apparent to those skilled in the art. Preferably, but not necessarily, the protein carrier will be foreign to the host animal in which antibodies against the Eimeria proteins are to be elicited.

Covalent coupling to the carrier molecule can be carried out using methods well known in the art, the exact choice of which will be dictated by the nature of the carrier molecule used. When the immunogenic carrier molecule is a protein, the proteins or fragments of the invention can be coupled, e.g., using water soluble carbodiimides such as dicyclohexylcarbodiimide, or glutaraldehyde.

Coupling agents such as these can also be used to cross-link the proteins and fragments to themselves without the use of a separate carrier molecule. Such cross-linking into protein or protein fragment aggregates can also increase immunogenicity.

Administration of an effective amount of the vaccines of this invention can protect poultry against infection by E. tenella. Monoclonal antibodies against the E. tenella antigens cross-react with E. acervulina and E. maxima in vitro, indicating that protection may also be conferred against these species. An effective dose of the proteins or protein fragments ranges from about 5 to about 50 micrograms/kg of body weight of the vaccinated animal. A dose of about 25-50 wg/kg is preferred. Initial vaccinations are preferably followed by booster vaccinations given from one to several weeks later. Multiple boosters may be administered. The dosages of such boosters generally range from about 5 to 50 pg/kg, preferably about 20-50 pg/kg. Standard routes of administration can be used such as subcutaneous, intradermal, intramuscular, oral, anal or in ovo administration.

The presentation of the coccidial antigens of the invention to the immune systems of fowl can also be achieved by cloning genes coding for the antigens into bacteria (e.g., E. coli or Salmonella) or into viruses (e.g., poxviruses or herpesviruses) and administering the live vector system or, when appropriate, its inactivated form to the birds orally, by injection or by other commonly used routes. Carbit et al. [in: Vaccines, 1987, Cold Spring Harbor Laboratory, pp. 68-71] have described the use of E. Coli, while Clements [Pathol. Immunopathol. Res. 6:137 (1987)] has described the use of Salmonella. Moss et al. [Ann. Rev. Immunol. 5:305 (1987)] have reviewed the use of viral vector systems employing recombinant poxviruses.

One kind of poxvirus, vaccinia virus, can be used to test the delivery of coccidial antigens in cell culture and in animals. For analytical studies, vaccinia virus has been found to be more efficient than fowlpox virus, another poxvirus carrier that can be used. This is because vaccinia virus multiplies more rapidly than the avian virus and has a host range that is not restricted to chicken cells. Large amounts of heterologous DNA can be inserted into the vaccinia viral genome without inhibiting viral maturation and infectivity [Smith et al., Gene 25:21 (1983)]. The insertion and expression of multiple heterologous genes using the virus elicits antibody production against expressed antigens in infected animals [Perkus et al., Science 229:981 (1985)].

The techniques used to produce recombinant vaccinia viruses can be readily adapted by routine procedures to fowipox or herpesvirus systems. A recombinant virus comprising a DNA having a nucleotide sequence encoding a protein of the present invention may be prepared by:
(a) inserting a DNA having a nucleotide sequence encoding the said protein into the genome of a virus without inhibiting viral maturation and infectivity:
(b) amplifying the said recombinant virus in a cell culture: and
(c) purifying the recombinant virus from the culture medium.

The use of recombinant viruses as carriers in vaccines against coccidiosis is especially advantageous in that vaccinated fowl develop immunity against both the coccidial antigen and the viral carrier (i.e., such vaccines are bivalent). The utility of such vaccines can be further enhanced by inserting additional genes into the carrier virus. For example, parts of the Newcastle disease viral genome can be inserted together with a coccidial antigen gene into a fowlpox virus, thereby conferring immunity against Newcastle disease, coccidiosis and fowlpox, all with a single vaccine.

The administration of the live vector vaccines of the invention can be carried out by numerous methods well known in the art. For example, the "stick" method commonly used to vaccinate poultry against fowlpox virus can be used. This method consists of sticking or pricking the skin of the wing web with a sharp needle dipped into the vaccine. The needle usually has an eye nearthe tip like a sewing machine needle which carries a drop of vaccine. Alternatively, the live vaccines can be injected subcutaneously or intradermally into the wing web or any other site.

The recombinant live vector vaccines can also be added to drinking water or even sprayed over chicks that are to be vaccinated. They can also be administered in feed, preferably after protective encapsulation [Bal- ancou et al., Nature 322:373 (1986)], or in ovo. In the latter method, the viral vaccines are injected directly into chicken embryos [Sharma, Avian Dis. 25:1155 (1985)].

### EXAMPLE

All references cited herein are hereby incorporated by reference in their entirety.

Unless otherwise specified, percentages given below for solids in solid mixtures, liquids in liquids, and solids in liquids are on a wt/wt, vol/vol and wt/vol basis, respectively.

### Purification of Merozoites

Merozoites of E. tenella were harvested from the ceca of 50 infected chickens (3 week old Hubbard Cross; Avian Services, Frenchtown, NJ) 5 days after infection with 50,000 of the above sporulated oocysts/bird. Similar chickens from other sources may be used. The ceca were removed and washed with phosphate buffered saline (PBS) for 15 minutes on a magnetic stirrer. The epithelial debris was partially removed by low speed centrifugation (50 x g), and the crude merozoites were recovered by centrifugation at 2,000 x g at 4°C for 10 minutes. The pellet was resuspended in Lysing Buffer (8.29 g/I NH₄CI, 0.372 g/I Na₂EDTA, 1.0 g/I KHC0₃, pH 7.6) and incubated on ice for 30 minutes. The merozoites were collected by centrifugation, washed once in PBS and passed over a column containing 1.0 g of spun nylon fiber (Scrub Nylon Fiber, Fenwall Laboratories, Deer- field, IL) in a separatory funnel. The merozoites were collected by centrifugation as before and frozen on dry ice for RNA isolation, or further purified in diethylaminoethyl cellulose (DEAE, Whatman DE52, Whatman Bio Systems, Inc., Clifton, NJ) for Western blot analysis.

For purification in DEAE cellulose, approximately 1 x 10⁹ merozoites were applied in PBS to a 10-ml bed volume column and eluted with PBS. The merozoites were recovered in the first 100 ml of flow-through, essentially free of red blood cells and other cellular debris.

### Immunoprecipitation of ¹²⁵1-Labeled Surface Proteins

The surface proteins of purified merozoites were labeled with ¹²⁵1 by the IODOGEN method (Pierce Chemical Co.) or by use of IODOBEADS (Pierce Chemical Co.). For the latter procedure, 4 IODOBEADS were washed 3 x with 0.2 M sodium phosphate, pH 7.5, and 1-3 mCi of ¹²⁵1-Na were added and incubated for 5 minutes at room temperature. Purified merozoites (3 x 10⁸ ) in 200 µl of PBS, pH 7.0, were added to the reaction vial, and the incubation was continued for 15 minutes. At the end of the incubation, phenylmethanesulfonyl fluoride (PMSF) was added to a final concentration of 5 mM.

The labeled merozoites were recovered from the incubation mixture by centrifugation at 12,000 x g for 30 seconds and solubilized in 1 ml of either 2% sodium dodecysulfate (SDS) or 1% Triton X-100 in PBS, pH 7.0. Insoluble material was removed by centrifugation for 3 minutes at 12,000 x g. The solubilized proteins were dialyzed against 3 liters of PBS, pH 7.0, at 4°C using a 3,500 molecular weight cutoff membrane to remove any residual free ¹²⁵1. The ¹²⁵1-labeled proteins (typically about 1.5 x 10⁸ cpm incorporated into protein) were stored at 4°C until used. The TCA precipitable radioactivity was typically in excess of 95% of the total radioactivity.

Rabbit antiserum against glutaraldehyde-fixed merozoites was prepared as follows:
Approximately 1 x 10⁹ purified merozoites were suspended in 1% gluteraldehyde in PBS and incubated at room temperature for 5 minutes. The fixed parasites were harvested by centrifugation at 2000 x g for 5 minutes, washed three times with PBS and resuspended in 1 ml PBS. New Zealand white rabbits were given multiple intradermal injections in the skin of the back with a total of 0.5 ml of the fixed parasite solution emulsified with 0.5 ml complete Freund's adjuvant. Rabbits received two booster injections containing the same parasite protein in incomplete Freund's adjuvant at two week intervals. Blood was harvested from the ear vein two weeks afterthe last boost and serum containing antibodies was obtained by centrifugation of coagulated blood samples for 15 minutes at 2500 x g.

Samples of labeled proteins for immunoprecipitation (5 µl, containing 5 x 10⁵ cpm) were diluted into 100 µl of IP buffer (0.25% NP-40, 20 mM Tris-HCI, pH 7.5, 0.15 M NaCI), pre-cleared by incubation for 20 minutes on ice with 5 µg of Staph-A protein (Pansorbin@, Calbiochem Corp., San Diego, CA), and incubated for several hours at 4°C with 5-10 µl of the rabbit anti-merozoite serum. The antibody complexes were collected by a second incubation with 5µg of Staph-A protein for 20 minutes on ice and centrifuged for 15 seconds in an Eppen- dorf centrifuge. The pellets were washed 4 times with IP buffer, and the labeled proteins immunoprecipitated by the antibody reagent were eluted from the complex by heating to 100°C for 5 minutes in SDS gel sample buffer (65 mM Tris pH 6.8, 0.5% SDS, 5% (3-mercaptoethanol, 10% glycerol, 0.1% Bromophenol blue). SDS PAGE was carried out as described by Laemmli [Nature 227:680 (1970)].

Results obtained with the rabbit antiserum were confirmed using immune chicken serum prepared as follows:
Chickens were immunized by repeated infection with viable sporulated oocysts of E. tenella (100,000 oocysts, given 3 times at 2 week intervals). Blood was harvested by cardiac puncture and the serum containing antibodies was separated from coagulated debris following centrifugation at 2500 x g for 5 minutes.

Comparison studies were carried out in which both the anti-merozoite rabbit serum and the immune chicken serum were used to immunoprecipitate (1) ¹²⁵1-surface-labeled Eimeria merozoite proteins and (2) the in vitro products of the translation of poly(A)-containing merozoite RNA. The precipitated proteins were then subjected to SDS PAGE and visualized by fluorography using standard fluorography techniques and reagents.

These studies showed that the many proteins from both sources were precipitated by both sera. Thus, either serum could be used to screen genetic recombinants expressing Eimeria proteins. For convenience, the rabbit anti-merozoite serum was used first in the screening procedures described below. However, immune chicken serum was used in parallel screening of the cDNA library as described below. This was essential for the identification of proteins likely to be important in the immune response to the infectious organism, because only the chicken serum was produced in response to challenge with live organisms. Only the immunized chickens were demonstrably resistant to such organisms.

To increase the specificity of the rabbit anti-merozoite serum for Eimeria proteins, antibody select was carried out on the sera essentially as described by Hall et al., supra. Briefly, antibodies specific for the precursor protein expressed by a recombinant phage clone (see below) were purified from the rabbit anti-merozoite serum as follows.

The positive phage was plated to high density and grown at 42°C for 3.5 hours. Expression of the fusion protein was induced by over layering the plate with a nitrocellulose filter saturated with 10 mM isopropylthio- galactoside (IPTG), and incubation was continued at 37°C for 6-8 hours. The antigen-loaded filters were washed in TBS (20 mM Tris HCI, pH 8.0, 150 mM NaCI) and incubated for 8-10 hours at 4°C with excess anti-merozoite serum which had been pre-absorbed with the E. coli host bacteria. The filters were washed 3 times with TBS to remove non-specific antibodies.

The antibodies specifically bound to the fusion protein on the filters were eluted with 2.0 ml of 0.1 M glycine, pH 2.6, 0.15 M NaCI (15 minutes at 20°C). The eluted antibodies were neutralized immediately with an equal volume 0.1 M Tris HCI, pH 8.0. The selected antibodies (hereinafter referred to as "antibody-select antibodies") were then used in the immunoprecipitation of surface-labeled merozoites or in vitro translation products, or as probes in Western blots of whole merozoite protein. Control sera were prepared using non-recombinant phage in the antibody-select procedure.

The results of Western blot and immunoprecipitation analyses using the antibody-select antibodies are shown in Fig. 2. The products of the immunoprecipitation of labeled proteins were visualized by fluorography as described by Bonneret al. [Eur. J. Biochem, 46:83 (1974)]. Numbers to the right of the figure show the positions of molecular weight marker proteins having the indicated sizes in kilodaltons.

Panel Aof Fig. 2 shows an immunoblot of total merozoite proteins probed with control (a) or antibody-select antibodies (b). Panel B shows ¹²⁵1-face-labeled merozoite proteins that had been immunoprecipitated with control (a), or antibody-select (b) antibodies.

### Isolation and In vitro Translation of Merozoite mRNA

Frozen merozoite pellets containing 1 x 10⁹ to 1 x 10¹⁰ organisms were thawed into 10 ml of TEL/SDS buffer (0.2 M Tris HCI, 0.1 M LiCI, 25 mM EDTA, 1% (w/v) sodium dodecyl sulfate (SDS), pH 8.8) containing 1 mM dithiothreitol (DTT) and 300 units of RNasin (Promega Biotec, Madison, WI) and homogenized with 10-12 strokes in a teflon-coated tissue homogenizer. Insoluble debris was separated by centrifugation in the cold at 3,000 x g. The supernatant fluid was extracted twice with phenol:chloroform:isoamyl alcohol (24:24:1,v/v) which had been equilibrated with the TEL buffer.

The aqueous phase was digested with 100 mg/ml proteinase K at 37°C for 30 minutes and reextracted with an equal volume of phenol:chloroform (1:1), and the nucleic acid was precipitated with two volumes of ethanol for 1 hour on dry ice, or overnight at -20°C. The pellet, after centrifugation at 10,000 x g for one hour, was resuspended in TE (10 mM Tris, pH 7.5, 2 mM EDTA) and spun through a 4 ml CsCI cushion (5.7 M CsCI, 0.1 M EDTA) at 150,000 x g for 20 hours at 15°C. The RNA pellet was reprecipitated from 0.2 M potassium acetate with 2.5 volumes of ethanol. This total RNA was passed once over oligo-dT cellulose to enrich for poly(A)⁺ RNA, as described by Maniatis, supra, page 197. Atypical yield of 1.9 mg of total RNA from 5 x 10⁹ merozoites contained approximately 20 µg of poly(A)⁺RNA.

Between 0.1 and 0.5 µg of mRNA was used to program in vitro protein synthesis in a nuclease-treated rabbit reticulocyte lysate (Amersham Corp., Arlington Heigths, IL or Promega Biotec) supplemented with 10-20 µCi of ³⁵S-Methionine per 20 µl of reaction mixture. The in vitro translation products were analyzed by immunoprecipitation followed by SDS PAGE and visualized by fluorography as described above, with the results shown in Fig. 2, Panel C.

Lane a of Panel C shows the complete mixture of products programmed by the poly (A)-containing merozoite RNA. Lane b, c and d show translation products immunoprecipitated by antibodies selected by a recombinant phage clone designated lambda 5-7 (see below; this clone expresses a gene encoding the Eimeria precursor protein), another phage clone reacting with anti-merozoite serum and a non-recombinant lambda gtll clone, respectively.

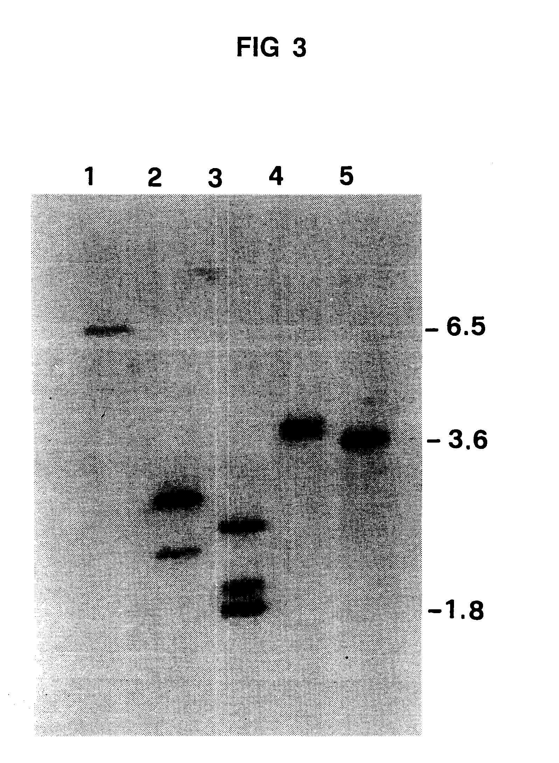

It should be noted that a major protein having an apparent molecular weight of about 30 kilodaltons can be seen in lanes a and b, Figure 2, Panel c. This protein is not present in the lane containing total merozoite proteins probed with antibody-select antibodies (Panel A, lane b), but a 23 kilodalton band can be seen in this gel (Panel A, lane b, arrow). A protein of 23 kilodaltons was also immunoprecipitated by the antibody-select antibodies from ¹²⁵1-labelled merozoite proteins as shown in Figure 3, panel B, lane b. These observations together suggest that the 30 kilodalton precursor protein may be processed by proteolytic cleavage in mature merozoites to the 23 kilodalton surface antigen.

### Preparation of a Merozoite cDNA Expression Library

Double-stranded cDNAwas synthesized from 6 µg of the merozoite poly (A)⁺RNAas described by Gubler et al., Gene 25:263 (1983), using reverse transcriptase (BRL, Gaithersburg, MD) to elongate from an oligo(dT) primer and RNase H (BRL) and E. coli DNA polymerase I (New England Biolabs, Beverly, MA) to synthesize the complementary strand. The double-stranded cDNAwas then blunt-ended with T4 DNA polymerase (BRL), and Eco Rllinkers (GGAATTCC, Collaborative Research Inc., Bedford, MA) were added after treatment with EcoRl methylase (New England Biolabs), following the manufacturers' protocols.

Following digestion with EcoRl, the cDNAs were fractionated in Biogel A-50M to remove excess linker molecules and cDNAs smaller than approximately 300 bp, as described by Huynh et al., infra. The cDNAwas then concentrated by precipitation from ethanol.

A library was prepared in Âgtll (Stratagene Cloning Systems, San Diego, CA) as described by Huynh et al., in D. Glover (ed.), DNA Cloning Vol. I: A Practical Approach, 1985, IRL Press, Washington, D.C., pp. 49-78. The EcoRl cDNAfragments were ligated to EcoRl digested, dephosphorylated λgtll arms (Stratagene Cloning Systems), and the resulting DNA was packaged into phage with the Gigapack@ kit (Stratagene Cloning Systems), following the manufacturer's protocol.

The resulting library was amplified by plating on Y1088 host cells. The percentage of recombinants was estimated from the ratio of blue to colorless plaques on X-gal plates (Maniatis, supra, page 24) in the presence of isopropyl thiogalactoside (IPTG, Sigma Chemical Co.) to be about 90%.

### Immunological Screening of the cDNA Library

The Âgtll merozoite cDNAexpression library was plated on Y1090 cells at a density of about 10,000 plaques per 150 mm plate. Six such plates were incubated for 3.5 hours at 42°C, overlayered with nitrocellulose filters previously soaked in 10 mM IPTG to induce the expression of the β-galactosidase fusion protein, and incubated for an additional 4-5 hours to overnight at 37°C. The filters were removed from the plates and subjected to several batchwise washes with TBS (20 mM Tris HCI, pH 8.0, 0.15 M NaCI). Nonspecific protein binding sites were blocked by incubation in 20% fetal calf serum (FCS) in TBS for one hour at room temperature.

The filters were then incubated for one hourwith rabbit anti-merozoite serum which had been preadsorbed with the Y1090 cells, at 1:100 dilution in TBS containing 20% calf serum. Nonspecific antibodies were removed in successive washes with TBS, one of which contained 0.1% NP-40. The filters were incubated with goat anti-rabbit peroxidase conjugate (BioRad, Richmond, CA) at 1:1000 dilution in TBS plus calf serum for one hour at room temperature. The color reaction was developed with 4-chloro-1-naphthol (BioRad) following the manufacturer's instructions.

Serum from immune chicks was also used for the screening. This serum was preadsorbed with Y1090 cells and used at the same dilution as the rabbit serum. Rabbit anti-chicken antibody was used as the secondary antibody, and goat anti-rabbit horseradish peroxidase conjugate was used as the detecting antibody. Single plaques were isolated in a secondary screen using the same reagents.

One clone, designated lambda 5-7, produced a protein that was strongly reactive with antibodies from the rabbit serum. Asecond isolate, 1-5 was identified by screening with immune chick serum, and proved to contain a cDNA insert of the same size as the 5-7 clone. The DNAsequence analysis indicated that these phage clones encoded the same merozoite antigen.

### Expression of the Lambda 5-7 cDNA in E. coli

A 1.2 kb insert from lambda 5-7 was isolated by EcoRl digestion and agarose gel electrophoresis [Maniatis et al., supra, pp. 157-170]. The EcoRl ends were repaired with Klenow polymerase in the presence of dATP and dTTP, and BamHl linkers (GGGATCCC) were ligated to both ends. The modified fragment was inserted into each of the three expression vectors pDS56/RBSII, pDS56/RBSII,-1 and pDS56/RBSII,-2 at the BamHl site. These three vectors are described below. Plasmids containing the inserts in both possible orientations were transformed as described by Mandel et al. [J. Mol. Biol. 53:159 (1970)] into E. coli strain M15 carrying the compatible plasmid pDMI.1. The E. coli strain M15 harboring plasmids pDS56/RBSII and pDMI.1 is described in European Patent Application, Publication No. 316 695.

### Plasmid Construction

Generally, plasmids pDS56/RBSII, -1 and -2 contain the regulatable promoter/operator element N-250PSN250P29 and the ribosomal binding sites RBSII, RBSII(-1) and RBSII(-2), respectively. These ribosomal binding sites were derived from the ribosomal binding site of the promoter P_{G25} of the E. coli phage T5 [European Patent Application, Publication No. 207 459] and were obtained via DNA synthesis.

Due to the high efficiency of expression, the above-mentioned plasmids can be maintained in E. coli cells only if the promoter/operator element is repressed by the binding of a lac repressor to the operator. The lac repressor is coded in the lacl gene. N250PSN250P29 can be repressed efficiently only when a sufficient number of repressor molecules is present in the cells. Therefore, the lacl^{q} allele, which contains a promoter mutant responsible for an increased expression of the repressor gene, was used. This lacl^{q} allele is present on the plasmid pDMI.1, as described below.

The pDMI.1 plasmid carries, in addition to the lac I gene, the neomycin phosphotransferase gene, which confers kanamycin resistance to the bacteria and which is used as the selection marker. pDMI.1 is compatible with the pDS56/RBSII, -1 and -2 plasmids. E. coli cells which are transformed with expression vectors PDS-56/RBSII, -1 and -2 must contain pDMI.1 to guarantee that the expression vector is held stable in the cells. Induction of this system is achieved by adding IPTG to the medium.

### Plasmid pDS56/RBSII

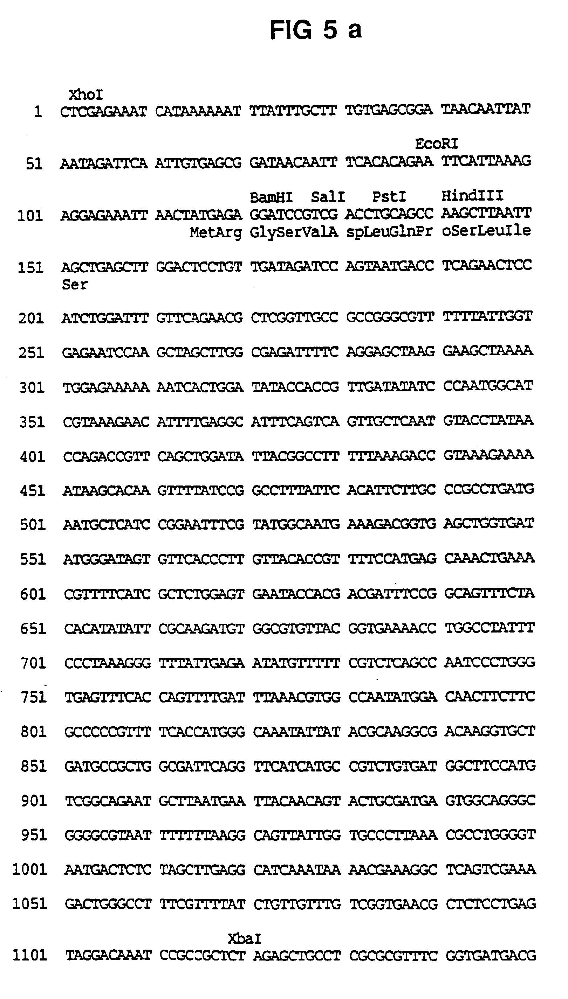

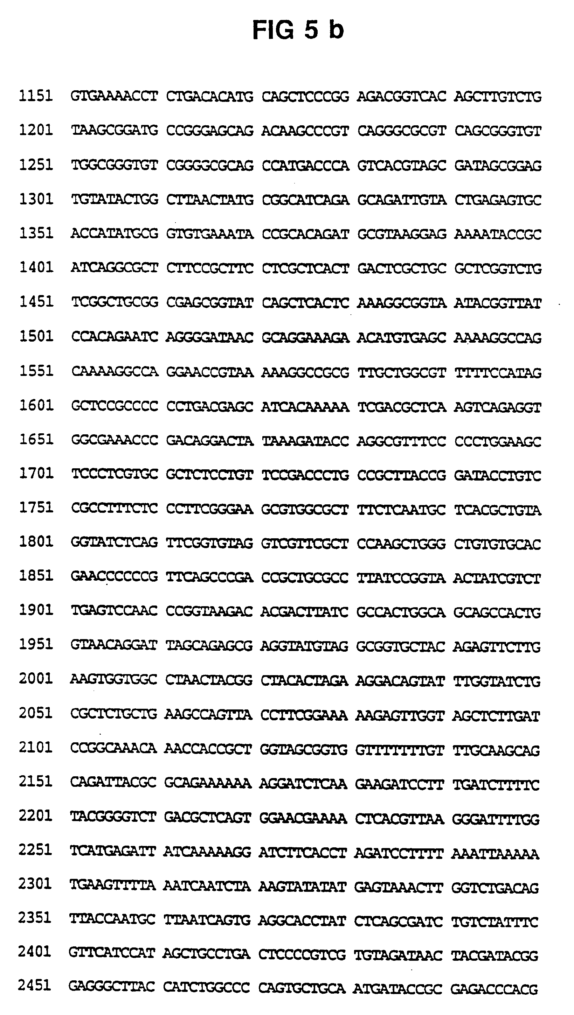

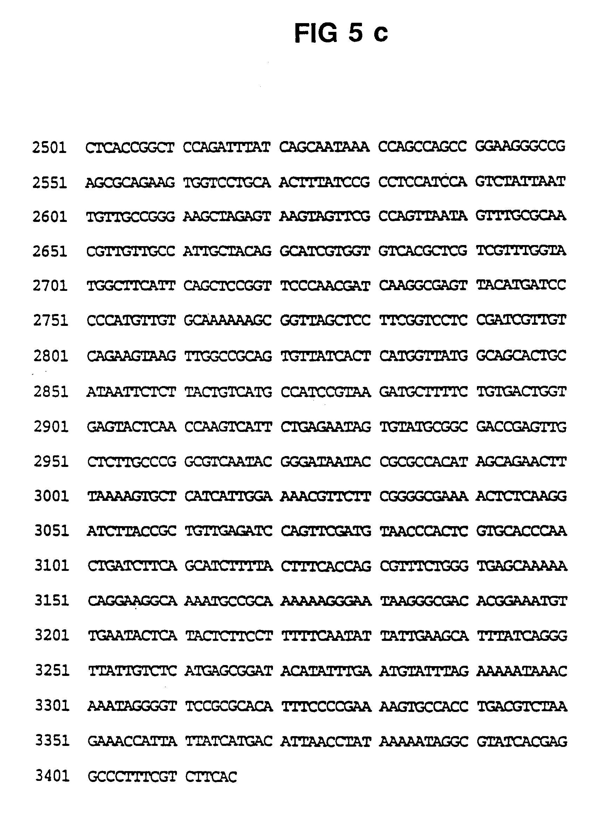

The part of pDS56/RBSII which lies between the restriction cleavage sites for Xbal and Xhol and which contains the replication region and the gene for β-lactamase (which confers ampicillin resistance to the cells) (Figs. 4 and 5) was derived originally from the plasmid pBR322 [Bolivar et al., Gene 2: 95-113 (1977); Sutcliffe, Cold Spring Harbor Symp. Quant. Biol. 43: 77-90 (1979)]. However, the gene for β-lactamase is modified by elimination of the cleavage sites for the restriction enzymes Hincll and Pstl. These alterations in the DNA sequence have no effect on the amino acid sequence of the β-lactamase. The remaining part of the plasmid carries the regulatable promoter/operator element N250PSOP29 followed by the ribosomal binding site RBSII, which is part of an EcoRl/BamHl fragment, cleavage sites for the restriction enzymes Sall, Pstl and Hindlll, the terminator to of E. coli phage lambda [Schwarz et al., Nature 272: 410-414 (1978)], the promoter-free gene of chloramphenicol acetyltransferase [Marcoli et al., FEBS Letters, 110: 11-14 (1980)] and the terminator T1 of the E. coli rrnB operon [Brosius et al., J. Mol. Biol. 148: 107-127 (1981)].

### Plasmid pDS56/RBSII(-1)

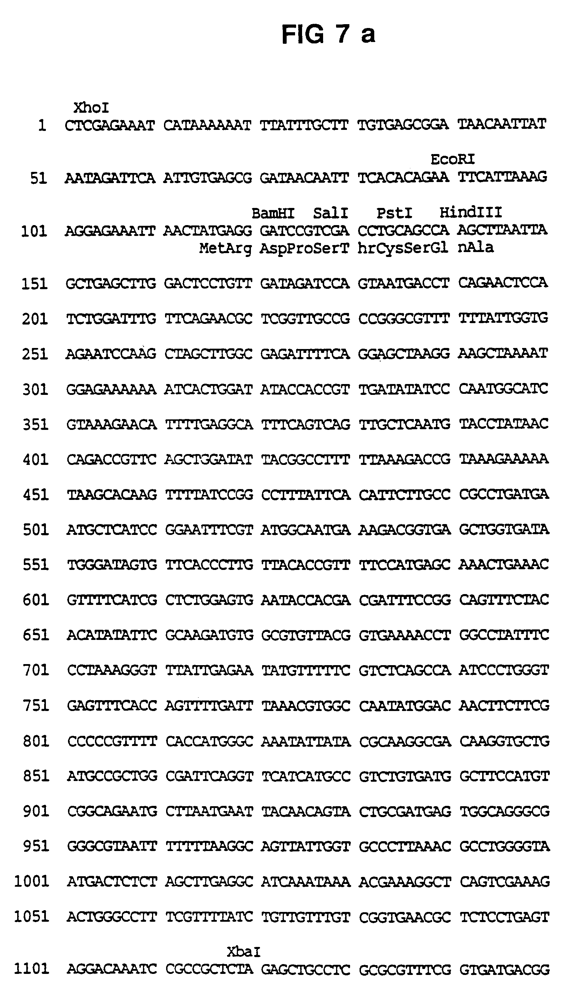

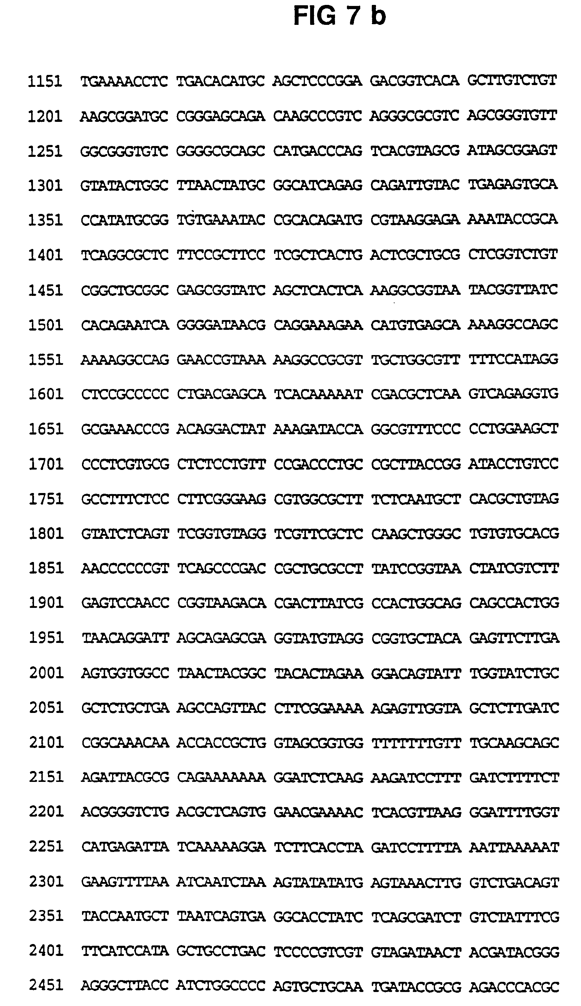

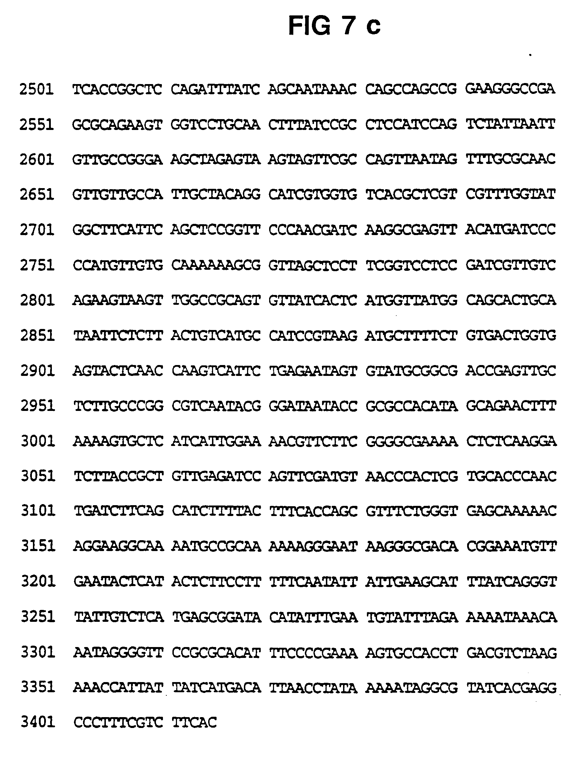

Plasmid pDS56/RBSII(-1) (Figs. 6 and 7) is similar to plasmid pDS56/RBSII but contains the ribosomal binding site RBSII(-1).

### Plasmid pDS56/RBSII(-2)

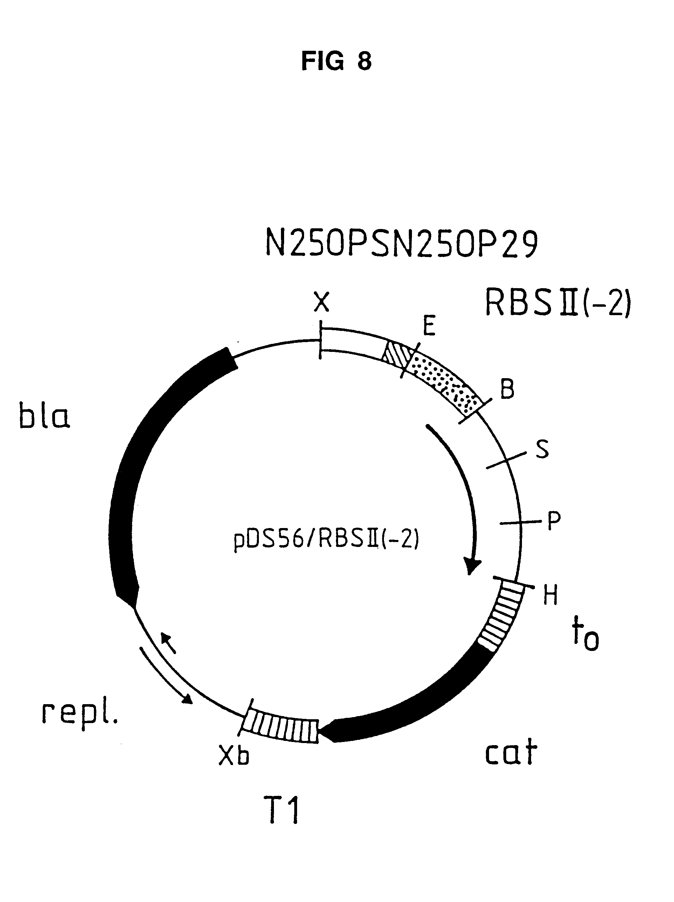

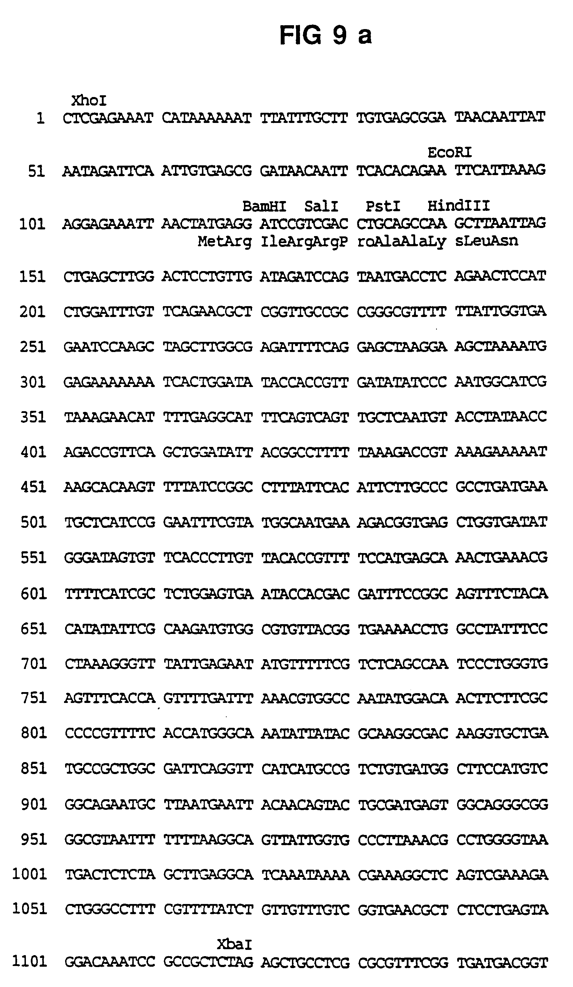

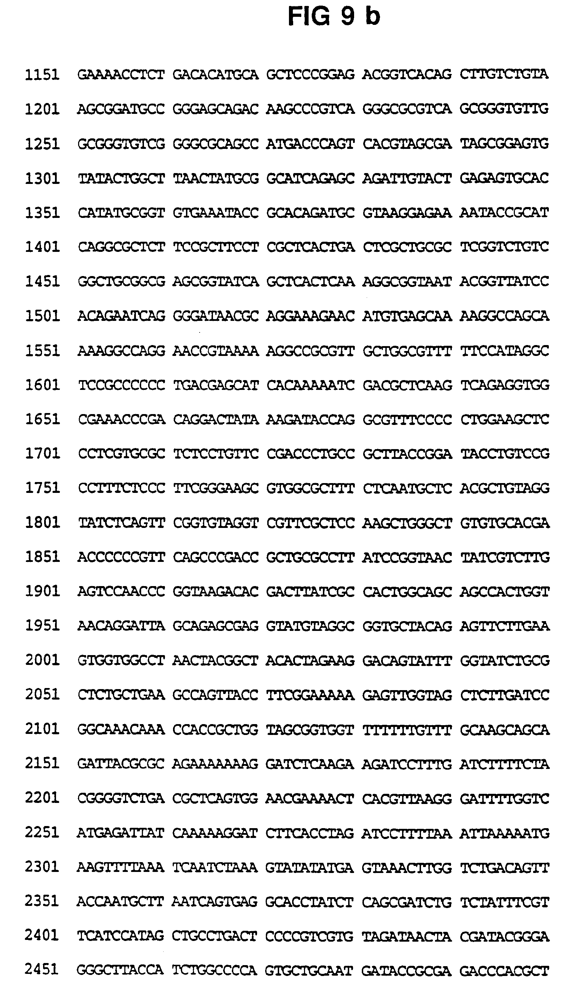

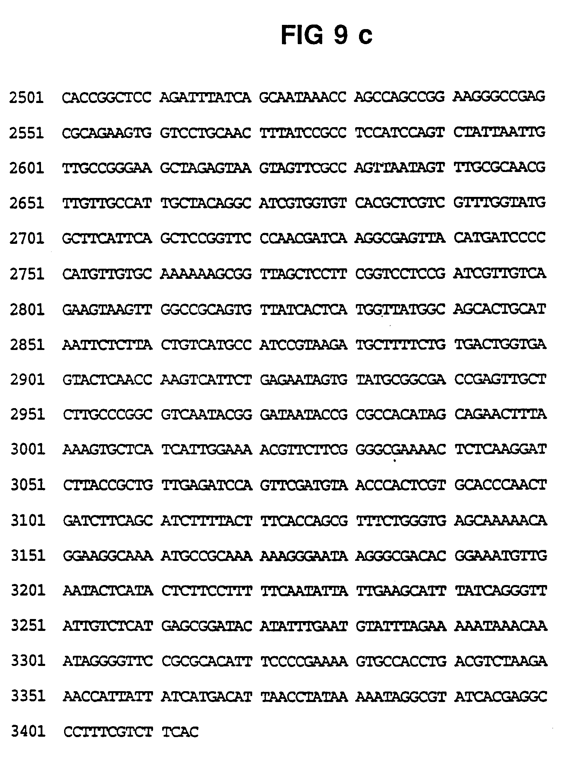

Plasmid pDS56/RBSII(-2) (Figs. 8 and 9) is similar to plasmid pDS56/RBSII but contains the ribosomal binding site RBSII(-2).

The difference in these three plasmids is that they differ by one nucleotide following the ATG start codon resulting in protein expression from all three potential reading frames.

### Plasmid pDMI.1

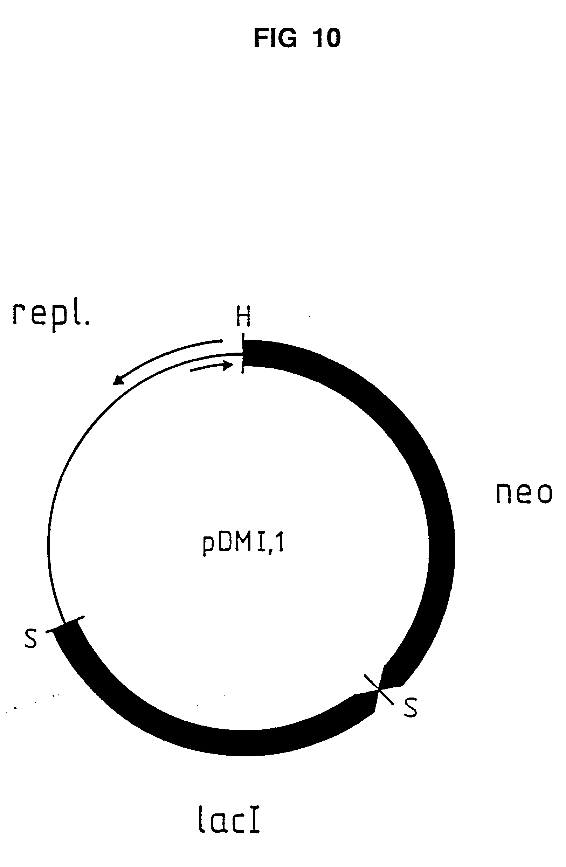

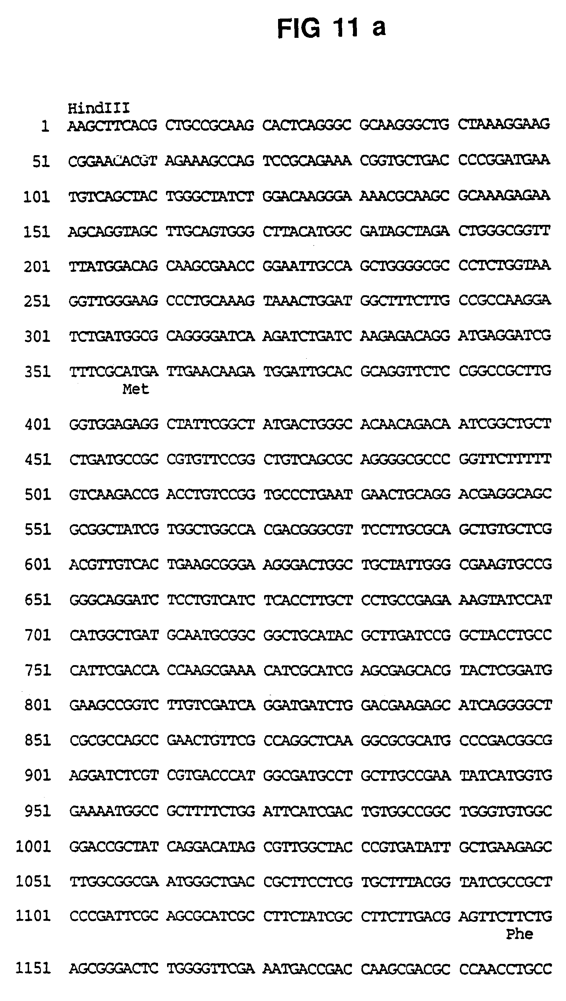

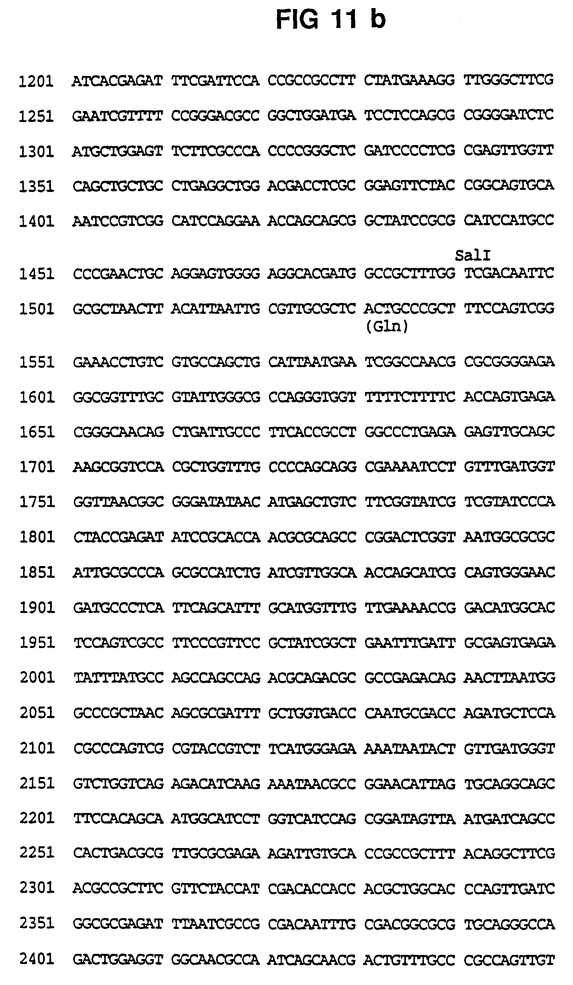

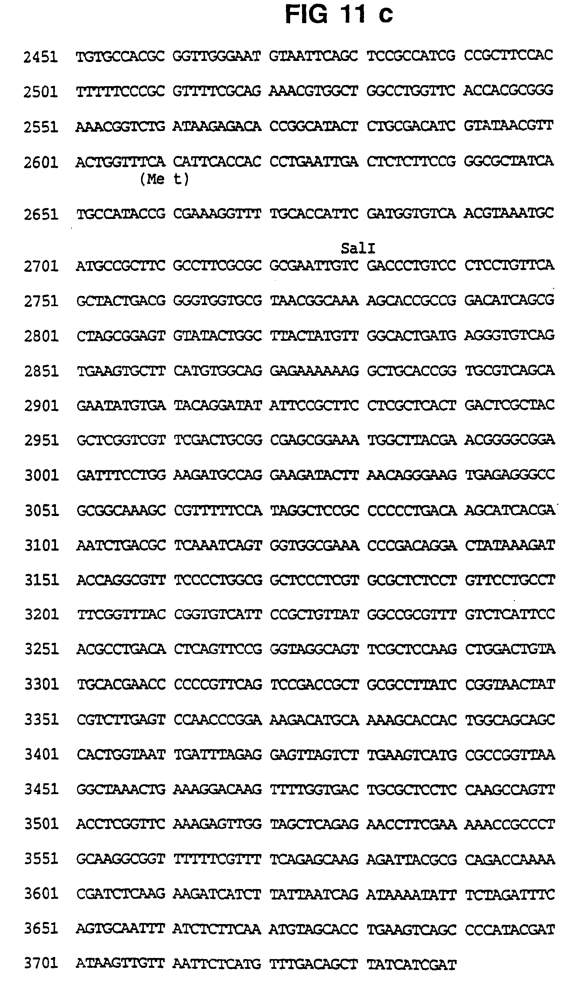

Plasmid pDMI.1 (Figs. 10 and 11) carries the gene for neomycin phosphotransferase from the transposon Tn5 [Beck et al., Gene 19: 327-336 (1982)], which confers kanamycin resistance to E. coli cells, and the lacl gene [Farabough, Nature 274: 765-769 (1978)] with the promoter mutation I^{q} [Calos, Nature 274: 762-765 (1978)], which codes for the lac repressor. Moreover, plasmid pDMI.1 contains a region of the plasmid pACYC184 [Chang and Cohen, J. Bacteriol. 134: 1141-1156 (1978)], which contains all information required for the replication and stable transmission to the daughter cells.

It should be understood that in addition to the above-described plasmid, any E. coli expression system is contemplated to be useful in this experiment.

The bacterial transformants were grown at 37°C in LB medium [Maniatis et al., supra, page 68] and expression of protein induced by addition of 1mM IPTG to the medium. After incubating for 1 hour, 1-ml samples were taken, and the cells in the samples were collected by centrifugation. The cell pellets were treated as described by Crowl et al., supra, and the lysates were subjected to SDS PAGE. Following electrophoresis, the proteins in the gels were either stained with Coomassie brilliant blue or transferred to nitrocellulose membranes for Western blot analysis [Towbin et al., Proc. Natl. Acad. Sci. USA 76:4350 (1979); Burnetti, Anal. Biochem. 112:195 (1981)], using the rabbit anti-merozoite serum as described above.

This analysis showed that the 1.2 kb cDNAmolecule in one orientation in all three reading frames produced a protein that migrated with an apparent molecular weight of about 30 kilodaltons and reacted with the antibodies from the rabbit anti-merozoite serum. This is consistent with the presence of stop codons in all three reading frames preceeding the ATG start codon at nucleotide 68 in the cDNA sequence, as shown in Figure 1.

### DNA Sequence Analysis

In general, small scale isolation of plasmid DNA from 1 ml of saturated overnight cultures was carried out using the procedure of Birnboim et al. [Nucleic Acids Research 7:1513 (1979)]. This procedure allows the isolation of a small quantity of DNA from a bacterial colony for analytical purposes. Larger amounts of plasmid DNA were prepared using 1-liter cultures following a standard protocol with cesium chloride centrifugation [Maniatis et al., supra, page 93].

The DNA sequence of the 1.2 kb EcoRl cDNAinsertfrom lambda 5-7 was determined as follows. The insert was digested with EcoRl, gel isolated, and ligated to the EcoRl digested pEV-vrf plasmid described by Crowl et al. [Gene 38:31 (1985)]. This plasmid was designated pEV/5-7 and was used to propagate the 1.2 kb cDNA insert for hybridization analysis (as described below) and in preliminary DNAsequence analysis by the method of Zagursky et al. [Gene Anal. Tech 2:89(1983)].

To determine the complete DNA sequence, the 1.2 kb cDNA insert was further subcloned into the M13, Mp18 and Mp19 single-stranded phage vectors using the Bio-Rad M13 Cloning and Sequencing Kit. The DNA sequence was determined by the dideoxy chain-termination method of Sanger et al. [Proc. Natl. Acad. Sci. USA 74: 5463 (1977)] using reagents and protocols provided with the Bio-Rad kit.

The complete nucleotide sequence of the 1.2 kb cDNAfrom lambda 5-7 including the 5' and 3' untranslated regions is shown in Fig. 1. Analysis of the sequence of a second isolate prepared as described above using immune chicken serum, designated 1-5, showed that this isolate contained the following additional nucleotide at the 5' end and lacks the EcoRl site of the 5-7 insert: 
AATTCGCCTTTNCGCTTGCACCCTTTGAGCTTCTTCTCGCCTGGAGACCTTGTGTCTGAAC .. (I-5)
AATTCGG .. (5-7)
 The remainder of the sequence of this second isolate is identical to that of lambda 5-7 from base number 8 to the beginning of the poly-A tract, except for nucleotide number 300, where a cytidine residue is found instead of a thymidine residue.

The cDNA sequence predicts an open reading frame extending from the ATG at position 68 to the TAA stop codon at position 668 encoding 200 amino acid residues as shown in Figure 1.

The theoretical size of 24 kilodaltons for a protein of 200 amino acids is slightly smaller than the estimated size of the primary translation product observed in the immunoprecipitation of merozoite mRNA (Figure 3, panel c, lane b) by the antibody-select reagent and the protein expressed from the cDNA in the E. coli expression vectors described above. However, this theoretical molecular weight is within the range of variation expected between theoretical molecular weights and molecular size determined by interpolation relative to molecular weight standards on SDS-PAGE.

Analysis of the deduced amino acid sequence of the protein encoded by the lambda 5-7 cDNA insert (Fig. 1) shows that the first twenty amino-terminal amino acid residues have an overall hydrophobic character, suggestive of a possible signal sequence.

### Hybridization Analysis

DNA was isolated from excysted, sporulated oocysts following treating with trypsin and bile and washing with PBS as follows:
The parasite material (approximately 1 x 10⁹ oocysts) was suspended in 20 ml of 0.5 M EDTA, pH 8.0, 0.5% Sarcosyl (Sigma, St. Louis, MO) and digested with proteinase K (Boehringer-Mannheim, BRD) at 0.1 µg/ml for 2 hours at 50°C, with RNase (10 µg/ml) for 1 hour at 37°C, and again with proteinase K for 1 hour at 50°C. The protein was removed with 2 extractions with phenol saturated with 20 mM Tris HCI, pH 7.5, 1 mM EDTA (TE), and one extraction with phenol/chloroform (1:1). The aqueous phase was dialysed extensively against TE and concentrated by ethanol precipitation. A typical yield of 0.4 mg DNA per 1 x 10⁶ oocysts was obtained.

The parasite DNAwas digested with various restriction endonucleases following the manufacturers' protocols and the resulting DNAfragments were resolved by electrophoresis at 40 V for 2.5 hours in 0.8% agarose in Loening Buffer (4.7 g NaH₂P0₄, 4.36 g Tris base, 0.372 g Na₂EDTA per liter, pH 7.6). The gel was treated with 0.25 M HCI for 30 minutes, and transferred to a Zeta-Probe membrane (Bio-Rad) in 0.4 M NaOH overnight. The filter was neutralized in 2 X SSC (pH 6.8) and baked for one hour at 80°C under vacuum.

The filterwas prehybridized for3 hours at 65°C in 7% SDS, 1% BSA(Boehringer, fraction V), 0.5 M NaHP0₄ buffer, pH 7.2. The 5-7 gene EcoRl insert was gel isolated following digestion of the pEv/5-7 plasmid, as described above, with EcoRl, and labeled by random-priming with Klenowfragment in the presence of32P-labeled deoxynucleotides. The labelled insert was separated from unincorporated nucleotides in Spin-Columns (Bio- Rad), denatured and added to the hybridization solution. Following incubation for 12 hours at 65°C, the filters were washed 3 times with 2 X SSC/0.1% SDS, and twice with 0.1 X SSC/0.1% SDS at 65°C. The genomic DNAfragments hybridizing to the probe were detected by autoradiography. Although the pEV/5-7 plasmid was used here, it is understood that any equivalent vector containing the 1.2 kb cDNA insert of the merozoite 5-7 gene would also perform in an acceptable manner.

The results of this analysis are shown in Fig. 3, where the results of digestion by Pvull (1), Hincll (2), Pstl (3), Sphl (4) or Sacl (5) can be seen.

Genomic DNAfragments of 6.5 and 3.6 kb were detected following digestion with Pvull and Sacl, in lanes 1 and 5, respectively. Since there are no sites for these enzymes in the cDNA done, the maximum size of the Eimeria gene can be estimated to be 3.6 kb. Digestion of genomic DNAwith EcoRl produced a 1.2 kb genomic fragment corresponding in size to the cDNAfragment. Double digestion with Hincll and EcoRl produced a 0.9 kb fragment predicted from the cDNA sequence flanked closely by EcoRl sites.

Three fragments were detected following digestion with Pstl (lane 3). Two Pstl sites are predicted from the cDNAsequence, which would produce an internal fragment of 305 bp and two joint fragments. The appearance of a third large Pstl fragment is probably the result of incomplete digestion at the internal Pstl sites.

The pattern of fragments produced by Sphl (lane 4), which also cuts twice in the cDNA, provides no definitive information. The small internal Sphl fragment predicted from the cDNAsequence could not have been detected in this gel.

In a Northern blot analysis [Alwine et al., Proc. Natl. Acad. Sci. USA 74: 5350 (1977)] of poly(A)-containing mRNA isolated from merozoites, the 1.2 kb cDNAfragment of the lambda 5-7 gene hybridized to a single mRNA species of approximately 1.3 kb in length. From the size correlation, it is apparent that the 5-7 clone, together with the 5' extension determined from the 1-5 isolate mentioned above, represents the full-length sequence of the cDNA, with the possible exception of the extreme 5' nucleotides.

Taken together, the foregoing observations are consistent with co-linearity of the cDNA and genomic sequences.

Many modifications and variations of this invention may be made without departing from its spirit and scope, as will become apparent to those skilled in the art. The specific embodiments described herein are offered by way of example only, and the invention is to be limited only by the terms of the appended claims.

## Claims (Claims for the following Contracting State(s) : AT, BE, CH, DE, DK, FR, GB, IT, Ll, LU, NL, SE)

1. A protein having one or more immunoreactive and/or antigenic determinants of an Eimeria merozoite surface antigen, which surface antigen has an apparent molecular weight of about 23 kilodaltons by SDS PAGE and is derived from a precursor protein having an apparent molecular weight of about 30 kilodaltons by SDS PAGE and which protein is encoded by the nucleotide sequence 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 or an equivalent sequence thereof, said protein being free of other Eimeria proteins.

2. The protein of claim 1 having the amino acid sequence 
M A K S M L S G I V F A G L V A A A A A
S S A N S A A N V S V L E S G P A V Q E
 
V P A R T V T A R L A K P L L L L S A L
A A T L A A A F L V L Q C F N X I S S N
N Q Q T S V R R L A A G G A C G D E E D
A D E G T S Q Q A S R R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 or a partial sequence thereof, such as the partial sequence lacking the first twenty amino acid residues in the amino acid sequence defined above, or a functional equivalent protein thereof, having an amino acid sequence which is related to the said amino acid sequence by deletions, insertions or substitutions without changing the immunological properties of the protein.

3. A DNA encoding a protein according to claim 1 or 2.

4. A DNA encoding a protein according to claim 1 or 2 having all or part of the nucleotide sequence 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTGATTGTGGCGAGG

ACAAGACAACACTTGAAGGATATCTTGGTAGTCAGCTCTTGCACAGGACGGAAAGACTGC

TAA
 or a functional equivalent thereof.

5. A recombinant vector comprising a DNA having a nucleotide sequence encoding a protein according to claim 1 or 2, which recombinant vector is capable of directing the expression of the said DNA in a compatible host organism.

6. A recombinant virus comprising a DNA having a nucleotide sequence encoding a protein according to claim 1 or 2, which recombinant virus is capable of directing the expression of the said DNA in a compatible host organism.

7. A transformed microorganism containing a recombinant vector comprising a DNA having a nucleotide sequence encoding a protein according to claim 1 or 2, which microorganism is capable of expressing the said DNA.

8. A protein according to claim 1 or 2 for the immunization of poultry against coccidiosis.

9. A method for producing a protein according to claim 1 or 2, which method comprises:
(a) culturing a microorganism containing a recombinant vector comprising a DNA having a nucleotide sequence encoding the said protein under conditions in which the DNA is expressed; and
(b) isolating the protein or fragment from the culture.

10. A method for producing a recombinant vector comprising a DNA having a nucleotide sequence encoding a protein according to claim 1 or 2, which method comprises:
(a) inserting a DNA having a nucleotide sequence encoding the said protein into a vector;
(b) replicating the said vector in a microorganism; and
(c) isolating the recombinant vector from the microorganism.

11. A method for producing a recombinant virus comprising a DNA having a nucleotide sequence encoding a protein according to claim 1 or 2, which method comprises:
(a) inserting a DNA having a nucleotide sequence encoding the said protein into the genome of a virus without inhibiting viral maturation and infectivity;
(b) amplifying the said recombinant virus in a cell culture; and
(c) purifying the recombinant virus from the culture medium.

12. A method for producing a transformed microorganism capable of producing a protein according to claim 1 or 2, which method comprises:
(a) transforming a microorganism with a recombinant vector comprising a DNA having a nucleotide sequence encoding the said protein; and
(b) growing the transformed microorganism in a fermentation broth.

13. A vaccine for protecting poultry against coccidiosis comprising a protein according to claim 1 or 2 and a physiologically acceptable carrier or adjuvant.

14. A vaccine for protecting poultry against coccidiosis containing a recombinant virus comprising a DNA having a nucleotide sequence encoding a protein according to claim 1 or 2, which recombinant virus is capable of directing the expression of the DNA in a compatible host organism, and a physiologically acceptable carrier or adjuvant.

15. The use of a protein according to claim 1 or 2 for the preparation of a vaccine capable of protecting poultry against coccidiosis.

## Claims (Claims for the following Contracting State(s) : GR, ES)

1. A process for the preparation of a protein having one or more immunoreactive and/or antigenic determinants of an Eimeria merozoite surface antigen, which surface antigen has an apparent molecular weight of about 23 kilodaltons by SDS PAGE and is derived from a precursor protein having an apparent molecular weight of about 30 kilodaltons by SDS PAGE and which protein is encoded by the nucleotide sequence 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 or an equivalent sequence thereof, said protein being free of other Eimeria proteins, which process comprises:
(a) culturing a transformed microorganism containing a recombinant vector comprising a DNA having a nucleotide sequence encoding the said protein under conditions in which the DNA is expressed; and
(b) isolating the protein from the culture.

2. A process according to claim 1 wherein the transformed microorganism contains a recombinant vector comprising a DNA sequence encoding a protein having the amino acid sequence 
M A K S M L S G I V F A G L V A A A A A
S S A N S A A N V S V L E S G P A V Q E
V P A R T V T A R L A K P L L L L S A L
A A T L A A A F L V L Q C F N X I S S N
N Q Q T S V R R L A A G G A C G D E E D
A D E G T S Q Q A S R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 or a partial sequence thereof, such as the partial sequence lacking the first twenty amino acid residues in the amino acid sequence defined above, or a functional equivalent protein thereof, having an amino acid sequence which is related to the said amino acid sequence by deletions, insertions or substitutions without changing the immunological properties of the protein.

3. A process for the preparation of a recombinant vector comprising a DNA having a nucleotide sequence encoding a protein as defined in claim 1 or 2, which process comprises:
(a) inserting a DNA having a nucleotide sequence encoding the said protein into a vector;
(b) replicating the said vector in a microorganism; and
(c) isolating the recombinant vector from the microorganism.

4. A process for the preparation of a recombinant virus comprising a DNA having a nucleotide sequence encoding a protein as defined in claim 1 or 2, which process comprises:
(a) inserting a DNA having a nucleotide sequence encoding the said protein into the genome of a virus without inhibiting viral maturation and infectivity;
(b) amplifying the said recombinant virus in a cell culture; and
(c) purifying the recombinant virus from the culture medium.

5. A process for the preparation of a transformed microorganism capable of producing a protein as defined in claims 1 or 2, which process comprises:
(a) transforming a microorganism with a recombinant vector comprising a DNA having a nucleotide sequence encoding the said protein; and
(b) growing the transformed microorganism in a fermentation broth.

6. A process for the preparation of a vaccine for the immunization of poultry against coccidiosis, which process comprises mixing a protein as defined in claim 1 or 2 with a pharmaceutically acceptable carrier.

7. The use of a protein as defined in claim 1 or 2 forthe preparation of a vaccine capable of protecting poultry against coccidiosis.

8. A DNAencoding a protein having one or more immunoreactive and/or antigenic determinants of an Eimeria merozoite surface antigen, which surface antigen has an apparent molecular weight of about 23 kilodaltons by SDS PAGE and is derived from a precursor protein having an apparent molecular weight of about 30 kilodaltons by SDS PAGE and which protein is free of other Eimeria proteins, which DNA sequence comprises all or part of the nucleotide sequence 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 or a functional equivalent thereof.

9. A recombinant vector comprising a DNA having a nucleotide sequence encoding a protein as defined in claim 1 or 2, which recombinant vector is capable of directing the expression of the said DNA in a compatible host organism.

10. The recombinant vector of claim 9 which is an E.coli vector.

11. A recombinant virus comprising a DNA having a nucleotide sequence encoding a protein as defined in claim 1 or 2, which recombinant virus is capable of directing the expression of the said DNA in a compatible host organism.

12. A transformed microorganism containing a recombinant vector comprising a DNA having a nucleotide sequence encoding a protein as defined in claim 1 or 2, which microorganism is capable of expressing the said DNA.

## Patentansprüche (Patentansprüche für folgende(n) Vertragsstaat(en) : AT, BE, CH, DE, DK, FR, GB, IT, LI, LU, NL, SE)

1. Ein Protein mit einer oder mehreren immunreaktiven und/oder antigenischen Determinante(n) eines Eimeria-Merozoitenoberflächen- antigens, wobei das Oberflächenantigen ein scheinbares Molekulargewicht von etwa 23 Kilodalton bei Polyakrylamidgelelektrophorese in SDS hat und von einem Vorgängermolekül mit einem scheinbaren Molekulargewicht von etwa 30 Kilodalton bei Polyakrylamidgelektrophorese in SDS abstammt und wobei das Protein von der Nukleotidsequenz kodiert wird: 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 oder einer äquivalenten Sequenz davon und das Protein frei ist von anderen Eimeriaproteinen.

2. Das Protein von Anspruch 1 mit der Aminosäuresequenz 
M A K S M L S G I V F A G L V A A A A A
S S A N S A A N V S V L E S G P A V Q E

V P A R T V T A R L A K P L L L L S A L
A A T L A A A F L V L Q C F N X I S S N
N Q Q T S V R R L A A G G A C G D E E D
A D E G T S Q Q A S R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 oder einer Teilsequenz davon, wie die Teilsequenz der die ersten zwanzig Aminosäurereste in der oben definierten Aminosäuresequenz fehlen, oder ein funktionell äquivalentes Protein davon mit einer Aminosäuresequenz, die sich auf besagte Aminosäuresequenz durch Deletionen, Insertionen oder Substitutionen bezieht ohne die immunologischen Eigenschaften des Proteins zu ändern.

3. Eine DNS die für ein Protein gemäss den Ansprüchen 1 oder 2 kodiert.

4. Eine DNS, die für ein Protein gemäss den Ansprüchen 1 oder 2 kodiert, mit der gesamten oder einem Teil der Nukleotidsequenz: 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTGATTGTGGCGAGG
 
ACAAGACAACACTTGAAGGATATCTTGGTAGTCAGCTCTTGCACAGGACGGAAAGACTGC

TAA
 oder einem funktionellen Äquivalent davon.

5. Ein rekominanter Vektor, der eine DNS mit einer Nukleotidsequenz, die für ein Protein gemäss Anspruch 1 oder 2 kodiert, umfasst, wobei der rekominante Vektor fähig ist, die Expression dieser besagten DNS in einem verträglichen Wirtsorganismus zu lenken.

6. Ein rekombinanter Virus, der eine DNS mit einer Nukleotidsequenz, die für ein Protein gemäss Anspruch 1 oder 2 kodiert, umfasst, wobei der rekombinante Virus fähig ist, die Expression dieser besagten DNS in einem verträglichen Wirtsorganismus zu lenken.

7. Ein transformierter Mikroorganismus, der einen rekombinanten Vektor enthält, der eine DNS mit einer Nukleotidsequenz, die für ein Protein nach Anspruch 1 oder 2 kodiert enthält, wobei der Mikroorganismus befähigt ist, besagte DNS zu exprimieren.

8. Ein Protein nach Anspruch 1 oder 2 zur Immunisierung von Geflügel gegen Kokzidiose.

9. Ein Verfahren zur Herstellung eines Proteins nach Anspruch 1 oder 2, welches
(a) die Kultivierung eines Mikroorganismus der einen rekombinanten Vektor, der eine DNS mit einer Nukleotidsequenz, die für besagtes Protein kodiert unter Bedingungen unter denen die DNS exprimiert wird, umfasst; und
(b) die Isolierung des Proteins oder Fragments aus der Kultur umfasst.

10. Ein Verfahren zur Herstellung eines rekombinanten Vektors der eine DNS umfasst mit einer Nukleotidsequenz die für ein Protein nach Anspruch 1 oder 2 kodiert, wobei das Verfahren:
(a) das Einsetzen einer DNS mit einer besagtes Protein kodierenden Nukleotidsequenz in einen Vektor;
(b) das Replizieren besagten Vektors in einem Mikroorganismus; und
(c) die Isolierung des rekombinanten Vektors aus dem Mikroorganismus umfasst.

11. Ein Verfahren zur Herstellung eines rekombinanten Virus der eine DNS mit einer Nukleotidsequenz, die für ein Protein nach Anspruch 1 oder 2 kodiert umfasst, wobei das Verfahren:
(a) das Einsetzen einer DNS mit einer Nukleotidsequenz die für besagtes Protein kodiert in das Genom eines Virus ohne die Reifung und die Übertragbarkeit des Virus zu hemmen;
(b) die Vervielfachung besagten rekombinanten Virus in einer Zellkultur; und
(c) die Reinigung des rekombinanten Virus aus dem Kulturmedien umfasst.

12. Ein Verfahren zur Herstellung eines transformierten Mikroorganismus der zur Herstellung eines Proteins nach Anspruch 1 oder 2 befähigt ist, wobei das Verfahren
(a) die Transformation des Mikroorganismus mit einem rekombinierten Vektor, der eine DNS mit einer Nukleotidsequenz, die besagtes Protein kodiert; und
(b) das Wachsen des transformierten Mikroorganismus in einer Fermentationsbrühe umfasst.

13. Ein Vakzin zum Schutz von Geflügel gegen Kokzidiose das ein Protein gemäss Anspruch 1 oder 2 und ein physiologisch verträglichen Träger oder Begleitstoff umfasst.

14. Ein Vakzin zum Schutz von Geflügel gegen Kokzidiose, das einen rekombinanten Vektor enthält, der eine DNS umfasst mit einer Nukleotidsequenz, die für ein Protein gemäss Anspruch 1 oder 2 kodiert, wobei der rekombinante Virus befähigt ist, die Expression der DNS in einem verträglichen Wirtsorganismus zu lenken, und einen physiologisch verträglichen Träger oder Begleitstoff.

15. Die Verwendung eines Proteins nach Anspruch 1 oder 2 zur Herstellung eines Vakzins das fähig ist, Geflügel gegen Kokzidiose zu schützen.

## Patentansprüche (Patentansprüche für folgende(n) Vertragsstaat(en) : GR, ES)

1. Ein Verfahren zur Herstellung eines Proteins mit einer oder mehreren immunreaktiven und/oder antigenischen Determinante(n) eines Eimeria-Merozoitenoberflächen-antigens, wobei das Oberflächenantigen ein scheinbares Molekulargewicht von etwa 23 Kilodalton bei Polyakrylamidgelelektrophorese in SDS hat und von einem Vorgängermolekül mit einem scheinbaren Molekulargewicht von etwa 30 Kilodalton bei Polyakrylamidgelektrophorese in SDS abstammt und wobei das Protein von der Nukleotidsequenz kodiert wird: 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 oder einer äquivalenten Sequenz davon und das Protein frei ist von anderen Eimeriaproteinen, wobei das Verfahren:
(a) die Kultivierung eines Mikroorganismus der einen rekombinanten Vektor, der eine DNS mit einer Nukleotidsequenz, die für besagtes Protein kodiert unter Bedingungen unter denen die DNS exprimiert wird, umfasst; und
(b) die Isolierung des Proteins oder Fragments aus der Kultur umfasst.

2. Ein Verfahren nach Anspruch 1, worin der transformierte Mikroorganismus einen rekombinanten Vektor enthält, der eine DNS-Sequenz umfasst, die für ein Protein mit der Aminosäuresequenz: 
M A K S M L S G I V F A G L V A A A A A
S S A N S A A N V S V L E S G P A V Q E
V P A R T V T A R L A K P L L L S A L
A A T L A A A F L V L Q C F N X I S S N
N Q Q T S V R R L A A G G A C G D E E D
A D E G T S Q Q A S R R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 oder einer Teilsequenz davon, wie die Teilsequenz der die ersten zwanzig Aminosäurereste in der oben definierten Aminosäuresequenz fehlen, oder ein funktionell äquivalentes Protein davon kodiert mit einer Aminosäuresequenz, die sich auf besagte Aminosäuresequenz durch Deletionen, Insertionen oder Substitutionen bezieht ohne die immunologischen Eigenschaften des Proteins zu ändern.

3. Ein Verfahren zur Herstellung eines rekombinanten Vektors der eine DNS umfasst mit einer Nukleotidsequenz die für ein wie in Anspruch 1 oder 2 definiertes Protein kodiert, wobei das Verfahren:
(a) das Einsetzen einer DNS mit einer besagtes Protein kodierenden Nukleotidsequenz in einen Vektor;
(b) das Replizieren besagten Vektors in einem Mikroorganismus; und
(c) die Isolierung des rekombinanten Vektors aus dem Mikroorganismus umfasst.

4. Ein Verfahren zur Herstellung eines rekombinanten Virus der eine DNS mit einer Nukleotidsequenz, die für ein wie in Anspruch 1 oder 2 definiertes Protein kodiert umfasst, wobei das Verfahren:
(a) das Einsetzen einer DNS mit einer Nukleotidsequenz die für besagtes Protein kodiert in das Genom eines Virus ohne die Reifung und die Übertragbarkeit des Virus zu hemmen;
(b) die Vervielfachung besagten rekombinanten Virus in einer Zellkultur; und
(c) die Reinigung des rekombinanten Virus aus dem Kulturmedien umfasst.

5. Ein Verfahren zur Herstellung eines transformierten Mikroorganismus der zur Herstellung eines wie in Anspruch 1 oder 2 definierten Protein befähigt ist, wobei das Verfahren
(a) die Transformation des Mikroorganismus mit einem rekombinierten Vektor, der eine DNS mit einer Nukleotidsequenz, die besagtes Protein kodiert; und
(b) das Wachsen des transformierten Mikroorganismus in einer Fermentationsbrühe umfasst.

6. Ein Verfahren zur Herstellung eines Vakzins zur Immunisierung von Geflügel gegen Kokzidiose, wobei das Verfahren das Mischen eines wie in Anspruch 1 oder2 definierten Proteins mit einem pharmazeutisch verträglichen Träger umfasst.

7. Die Verwendung eines wie in Anspruch 1 oder 2 definierten Proteins zur Herstellung eines Vakzins das fähig ist, Geflügel gegen Kokzidiose zu schützen.

8. Eine DNS, die für ein Protein kodiert mit einer oder mehreren immunreaktiven und/oder antigenischen De- terminante(n) eines Eimeria-Merozoitenoberflächen-antigens, wobei das Oberflächenantigen ein scheinbares Molekulargewicht von etwa 23 Kilodalton bei Polyakrylamidgelelektrophorese in SDS hat und von einem Vorgängermolekül mit einem scheinbaren Molekulargewicht von etwa 30 Kilodalton bei Polyakrylamidgelektrophorese in SDS abstammt und wobei die DNS-Sequenz die gesamte oder Teile der Nukleotidsequenz: 
ATGGCTAAGTCTATGCTTTCTGGAATTGTTTTTGCTGGTCTTGTTGCTGCTGCAGCGGCC
AGTTCGGCCAACAGCGCCGCCAACGTCTCCGTTTTGGAGAGTGGGCCCGCTGTGCAGGAA
GTGCCAGCGCGCACGGTCACAGCTCGCCTGGCGAAGCCTTTGCTGCTTCTTTCTGCTCTT
GCTGCGACTTTGGCAGCAGCTTTCCTCGTTTTGCAATGCTTCAACACCATCTCCAGCAAC
AACCAGCAAACCAGCGTCAGGAGACTGGCCGCCGGAGGTGCATGCGGAGATGAGGAAGAT
GCAGATGAGGGAACTTCACAGCAGGCCAGCCGGAGGAGGAGAAAACCTGATACCCCTGCA
GCAGATAAATACGATTTTGTTGGCGGAACTCCAGTTTCGGTCACTGAGCCGAATGTTGAT
GAAGTCCTTATCCAAATTAGAAATAAACAAATCTTTTTGAAGAACCCATGGACTGGACAA
GAAGAACAAGTTCTAGTACTGGAACGACAAAGTGAAGAACCCATTCTGATTGTGGCGAGG
ACAAGACAACACTTGAAGGATATCTTGGTAGTCAGCTCTTGCACAGGACGGAAAGACTGC
TAA
 umfasst oder ein funktionelles Äquivalent davon.

9. Ein rekombinanter Vektor, der eine DNS mit einer Nukleotidsequenz, die für ein wie in Anspruch 1 oder 2 definiertes Protein kodiert, umfasst, wobei der rekominante Vektor fähig ist, die Expression dieser besagten DNS in einem verträglichen Wirtsorganismus zu lenken.

10. Der rekombinante Vektor von Anspruch 9 der ein E. coli-Vektor ist.

11. Ein rekombinanter Virus, der eine DNS mit einer Nukleotidsequenz, die für ein wie in Anspruch 1 oder 2 definiertes Protein kodiert, umfasst, wobei der rekombinante Virus fähig ist, die Expression dieser besagten DNS in einem verträglichen Wirtsorganismus zu lenken.

12. Ein transformierter Mikroorganismus, der einen rekombinanten Vektor enthält, der eine DNS mit einer Nukleotidsequenz, die für ein wie in Anspruch 1 oder 2 definiertes Protein kodiert enthält, wobei der Mikroorganismus befähigt ist, besagte DNS zu exprimieren.

## Revendications (Revendications pour l'(les) Etat(s) contractant(s) suivant(s) : suivants : AT, BE, CH, DE, DK, FR, GB, IT, LU, LI, NL, SE)

1. Protéine ayant un ou plusieurs déterminants immunoréactifs et/ou antigéniques d'un antigène d'enveloppe de mérozoite d'Eimeria, lequel antigène d'enveloppe a une masse moléculaire apparente d'environ 23 kilodaltons par PAGE au SDS et est dérivé d'une protéine précurseur ayant une masse moléculaire apparente d'environ 30 kilodaltons par PAGE au SDS et laquelle protéine est codée par la séquence nucléotidique 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 ou une séquence équivalente à celle-ci, la dite protéine étant exempte d'autres protéines d'Eimeria.

2. Protéine selon la revendication 1, ayant la séquence d'acides aminés 
M A K S M L S G I V F A G L V A A A A A
S S A N S A A N V S V L E S G P A V Q E
V P A R T V T A R L A K P L L L L S A L
A A T L A A A F L V L Q C F N X I S S N
N Q Q T S V R R L A A G G A C G D E E D
A D E G T S Q Q A S R R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 ou une séquence partielle de celle-ci, telle que la séquence partielle n'ayant pas les premiers vingt résidus d'acides aminés dans la séquence d'acides aminés définie ci-dessus, ou une protéine en constituant un équivalent fonctionnel, ayant une séquence d'acides aminés qui est apparentée à la dite séquence d'acides aminés par des délétions, des insertions ou des substitutions, sans changement des propriétés im- munologiques de la protéine.

3. ADN codant pour une protéine selon l'une des revendications 1 ou 2.

4. ADN codant pour une protéine selon l'une des revendications 1 ou 2, ayant tout ou partie de la séquence nucléotidique 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTGATTGTGGCGAGG

ACAAGACAACACTTGAAGGATATCTTGGTAGTCAGCTCTTGCACAGGACGGAAAGACTGC

TAA
 ou un équivalent fonctionnel de celle-ci.

5. Vecteur recombinant comprenant un ADN ayant une séquence nucléotidique codant pour une protéine selon l'une des revendications 1 ou 2, lequel vecteur recombinant est apte à diriger l'expression du dit ADN dans un organisme hôte compatible.

6. Virus recombinant comprenant un ADN ayant une séquence nucléotidigue codant pour une protéine selon l'une des revendications 1 ou 2, lequel virus recombinant est apte à diriger l'expression du dit ADN dans un organisme hôte compatible.

7. Microorganisme transformé contenant un vecteur recombinant comprenant un ADN ayant une séquence nucléotidique codant pour une protéine selon l'une des revendications 1 ou 2, lequel microorganisme est apte à exprimer le dit ADN.

8. Protéine selon l'une des revendications 1 ou 2 pour l'immunisation de la volaille contre la coccidiose.

9. Procédé pour la production d'une protéine selon l'une des revendications 1 ou 2, lequel procédé comprend:
(a) la mise en culture d'un microorganisme contenant un vecteur recombinant comprenant un ADN ayant une séquence nucléotidique codant pour la dite protéine dans les conditions dans lesquelles l'ADN est exprimé; et
(b) l'isolement de la protéine ou du fragment de la culture.

10. Procédé pour la production d'un vecteur recombinant comprenant un ADN ayant une séquence nucléotidique codant pour une protéine selon l'une des revendications 1 ou 2, lequel procédé comprend:
(a) l'insertion d'un ADN ayant une séquence nucléotidique codant pour la dite protéine dans un vecteur;
(b) la réplication du dit vecteur dans un microorganisme; et
(c) l'isolement du vecteur recombinant d'avec le microorganisme.

11. Procédé pour la production d'un virus recombinant comprenant un ADN ayant une séquence nucléotidique codant pour une protéine selon l'une des revendications 1 ou 2, lequel procédé comprend:
(a) l'insertion d'un ADN ayant une séquence nucléotidique codant pour la dite protéine dans le génome d'un virus sans inhiber la maturation et l'infectivité virales;
(b) l'amplification du dit virus recombinant dans une culture cellulaire; et
(c) la purification du virus recombinant d'avec le milieu de culture.

12. Procédé pour la production d'un microorganisme transformé apte à produire une protéine selon l'une des revendications 1 ou 2, lequel procédé comprend:
(a) la transformation d'un microorganisme avec un vecteur recombinant comprenant un ADN ayant une séquence nucléotidique codant pour la dite protéine; et
(b) la croissance du microorganisme transformé dans un bouillon de fermentation.

13. Vaccin pour la protection de la volaille contre la coccidiose comprenant une protéine selon l'une des revendications 1 ou 2 et un support ou adjuvant physiologiquement acceptable.

14. Vaccin pour la protection de la volaille contre la coccidiose contenant un virus recombinant comprenant un ADN ayant une séquence nucléotidique codant pour une protéine selon l'une des revendications 1 ou 2, lequel virus recombinant est apte à diriger l'expression de l'ADN dans un organisme hôte compatible, et un support ou adjuvant physiologiquement acceptable.

15. Utilisation d'une protéine selon l'une des revendications 1 ou 2 pour la préparation d'un vaccin apte à protéger la volaille contre la coccidiose.

## Revendications (Revendications pour l'(les) Etat(s) contractant(s) suivant(s) : suivants : GR, ES)

1. Procédé pour la préparation d'une protéine ayant un ou plusieurs déterminants immunoréactifs et/ou antigéniques d'un antigène d'enveloppe de mérozoite d'Eimeria, lequel antigène d'enveloppe a une masse moléculaire apparente d'environ 23 kilodaltons par PAGE au SDS et est dérivé d'une protéine précurseur ayant une masse moléculaire apparente d'environ 30 kilodaltons par PAGE au SDS et laquelle protéine est codée par la séquence nucléotidique 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 ou une séquence équivalente à celle-ci, la dite protéine étant exempte d'autres protéines d'Eimeria, lequel procédé comprend:
(a) la culture d'un microorganisme transformé contenant un vecteur recombinant comprenant un ADN ayant une séquence nucléotidique codant pour la dite protéine dans des conditions dans lesquelles l'ADN est exprimé; et
(b) l'isolement de la protéine d'avec la culture.

2. Procédé selon la revendication 1, dans lequel le microorganisme transformé contient un vecteur recombinant comprenant une séquence d'ADN codant pour une protéine ayant la séquence d'acides aminés 
M A K S M L S G I V F A G L V A A A A A
S S A N S A A N V S V L E S G P A V Q E
V P A R T V T A R L A K P L L L L S A L
A A T L A A A F L V L Q C F N X I S S N
N Q Q T S V R R L A A G G A C G D E E D
A D E G T S Q Q A S R R R R K P D T P A
A D K Y D F V G G T P V S V T E P N V D
E V L I Q I R N K Q I F L K N P W T G Q
E E Q V L V L E R Q S E E P I L I V A R
T R Q H L K D I L V V S S C T G R K D C
 ou une séquence partielle de celle-ci, telle que la séquence partielle n'ayant pas les premiers vingt résidus d'acides aminés dans la séquence d'acides aminés définie ci-dessus, ou une protéine en constituant un équivalent fonctionnel, ayant une séquence d'acides aminés qui est apparentée à la dite séquence d'acides aminés par des délétions, des insertions ou des substitutions, sans changement des propriétés im- munologiques de la protéine.

3. Procédé pour la préparation d'un vecteur recombinant comprenant un ADN ayant une séquence nucléotidique codant pour une protéine selon l'une des revendications 1 ou 2, lequel procédé comprend:
(a) l'insertion d'un ADN ayant une séquence nucléotidique codant pour la dite protéine dans un vecteur;
(b) la réplication du dit vecteur dans un microorganisme; et
(c) l'isolement du vecteur recombinant d'avec le microorganisme.

4. Procédé pour la préparation d'un virus recombinant comprenant un ADN ayant une séquence nucléotidique codant pour une protéine selon l'une des revendications 1 ou 2, lequel procédé comprend:
(a) l'insertion d'un ADN ayant une séquence nucléotidique codant pour la dite protéine dans le génome d'un virus sans inhiber la maturation et l'infectivité virales;
(b) l'amplification du dit virus recombinant dans une culture cellulaire; et
(c) la purification du virus recombinant d'avec le milieu de culture.

5. Procédé pour la préparation d'un microorganisme transformé apte à produire une protéine selon l'une des revendications 1 ou 2, lequel procédé comprend:
(a) la transformation d'un microorganisme avec un vecteur recombinant comprenant un ADN ayant une séquence nucléotidique codant pour la dite protéine; et
(b) la croissance du microorganisme transformé dans un bouillon de fermentation.

6. Procédé pour la préparation d'un vaccin pour l'immunisation de la volaille contre la coccidiose, lequel procédé comprend le mélange d'une protéine selon l'une des revendications 1 ou 2 avec un support phar- maceutiquement acceptable.

7. Utilisation d'une protéine selon l'une des revendications 1 ou 2 pour la préparation d'un vaccin apte à protéger la volaille contre la coccidiose.

8. ADN codant pour une protéine ayant un ou plusieurs déterminants immunoréactifs et/ou antigéniques d'un antigène d'enveloppe de mérozoïte d'Eimeria, lequel antigène d'enveloppe a une masse moléculaire apparente d'environ 23 kilodaltons par PAGE au SDS et est dérivé d'une protéine précurseur ayant une masse moléculaire apparente d'environ 30 kilodaltons par PAGE au SDS et laquelle protéine est codée par la séquence nucléotidique 
ATGGCTAAGTCTATGCTTTCTGGAATTGTTTTTGCTGGTCTTGTTGCTGCTGCAGCGGCC

AGTTCGGCCAACAGCGCCGCCAACGTCTCCGTTTTGGAGAGTGGCCCGCTGTGCAGGAA

GTGCCAGCGCGCACGGTCACAGCTCGCCTGGCGAAGCCTTTGCTGCTTCTTTCTGCTCTT

GCTGCGACTTTGGCAGCAGCTTTCCTCGTTTTGCAATGCTTCAACACCATCTCCAGCAAC

AACCAGCAAACCAGCGTCAGGAGACTGGCCGCCGGAGGTGCATGCGGAGATGAGGAAGAT

GCAGATGAGGGAACTTCACAGCAGGCCAGCCGGAGGAGGAGAAAACCTGATACCCCTGCA

GCAGATAAATACGATTTTGTTGGCGGAACTCCAGTTTCGGTCACTGAGCCGAATGTTGAT

GAAGTCCTTATCCAAATTAGAAATAAACAAATCTTTTTGAAGAACCCATGGACTGGACAA

GAAGAACAAGTTCTAGTACTGGAACGACAAAGTGAAGAACCCATTCTCATTGTGGCGAGG

ACAAGACAACACTTGAAGGATATCTTGGTAGTCAGCTCTTGCACAGGACGGAAAGACTGC

TAA
 ou un équivalent fonctionnel de celle-ci.

9. Vecteur recombinant comprenant un ADN ayant une séquence nucléotidique codant pour une protéine selon l'une des revendications 1 ou 2, lequel vecteur recombinant est apte à diriger l'expression du dit ADN dans un organisme hôte compatible.

10. Vecteur recombinant selon la revendication 9, qui est un vecteur d'E. coli.

11. Virus recombinant comprenant un ADN ayant une séquence nucléotidique codant pour une protéine selon l'une des revendications 1 ou 2, lequel virus recombinant est apte à diriger l'expression du dit ADN dans un organisme hôte compatible.

12. Microorganisme transformé contenant un vecteur recombinant comprenant un ADN ayant une séquence nucléotidique codant pour une protéine selon l'une des revendications 1 ou 2, lequel microorganisme est apte à exprimer le dit ADN.
